# EUROPÄISCHE PATENTANMELDUNG

(11) **EP 4 660 630 A2**
(43) Veröffentlichungstag der Anmeldung: **10.12.2025**
(21) Anmeldenummer: 25203773.4
(22) Anmeldetag: 14.07.2022
(51) Int. Cl.: G01N 33/68

(54) **VISKOSIMETER UND VERFAHREN ZUR BESTIMMUNG EINER KONZENTRATION EINER KOMPONENTE IN EINEM FLUID MIT EINEM SOLCHEN VISKOSIMETER**

(30) Priorität: 28.07.2021 EP 21188210
(62) Teilanmeldung aus: 22184986.2
(71) Anmelder: Levitronix GmbH, 8048 Zürich (CH)
(72) Erfinder: Steinert, Daniel Dr., 8180 Bülach (CH); Nussbaumer, Thomas Dr., 8049 Zürich (CH)
(74) Vertreter: IPS Irsch AG

(57) **Zusammenfassung**

Es wird ein Konzentrationsmessgerät zur Inline Bestimmung einer Konzentration einer Komponente in einem Fluid vorgeschlagen, mit einer Antriebsvorrichtung (50) und mit einer Messvorrichtung (10), wobei die Messvorrichtung (10) ein Messgehäuse (20) mit einem Einlass (21) und mit einem Auslass (22) für das Fluid umfasst, sowie eine Messkammer (23), in welcher ein Rotor (3) mit einem ring- oder scheibenförmigen magnetisch wirksamen Kern (31) vorgesehen ist, wobei die Antriebsvorrichtung (50) ein Antriebsgehäuse (60) umfasst, in welchem ein Stator (6) angeordnet ist, welcher mit dem Rotor (3) als elektromagnetischer Drehantrieb (90) zusammenwirkt, wobei der Stator (6) als Lager- und Antriebsstator ausgestaltet ist, mit welchem der Rotor (3) im Betriebszustand berührungslos magnetisch um eine axiale Richtung (A) antreibbar ist, und mit welchem der Rotor (3) berührungslos magnetisch bezüglich des Stators (6) lagerbar ist, und wobei im Antriebsgehäuse (60) ferner eine Kontrolleinrichtung (8) für die Ansteuerung des Stators (6) vorgesehen ist, dadurch gekennzeichnet, dass die das Konzentrationsmessgerät eine Speichereinheit (40) aufweist, in welcher Kalibrierungsdaten für die Messvorrichtung (10) gespeichert sind, und das Konzentrationsmessgerät die Konzentration einer Komponente des Fluids anhand einer Betriebsgrösse des elektromagnetischen Drehantriebs (90) bestimmt. Ferner wird ein Verfahren zur Inline Bestimmung einer Konzentration einer Komponente in einem Fluid vorgeschlagen.

## Beschreibung

Die Erfindung betrifft ein Konzentrationsmessgerät zur Inline Bestimmung der Konzentration einer Komponente in einem Fluid, eine Einmalvorrichtung für ein solches Konzentrationsmessgerät, sowie ein Verfahren zur Bestimmung einer Konzentration einer Komponente in einem Fluid gemäss dem Oberbegriff des unabhängigen Patentanspruchs der jeweiligen Kategorie.

Die Viskosität eines Fluids ist im Allgemeinen ein Mass für die innere Reibung in einem Fluid und stellt in vielen Fällen eine wichtige Grösse dar, anhand derer Eigenschaften oder der Zustand eines Fluids untersucht werden können. Die dynamische Viskosität, die üblicherweise mit dem Buchstaben η bezeichnet wird, kann als ein Mass für die Zähigkeit eines Fluids angesehen werden. Ihre SI-Einheit ist 1 Poise, was 0.1 Pa • s entspricht. Neben der dynamischen Viskosität wird häufig auch die auf die Dichte des Fluids normierte kinematische Viskosität, die üblicherweise mit dem Buchstaben v bezeichnet wird, als Kenngrösse für ein Fluid verwendet. Die kinematische Viskosität ist also der Quotient aus der dynamischen Viskosität und der Dichte des Fluids. Im Rahmen dieser Anmeldung ist dem üblichen Sprachgebrauch folgend unter dem Begriff "Viskosität" die dynamische Viskosität zu verstehen.

Für viele Prozesse, beispielsweise in der Biotechnologie oder in der Pharmaindustrie ist es oft notwendig, die Viskosität eines Fluids zu kennen oder auch zu überwachen, weil Änderungen in der Viskosität erheblichen Einfluss auf andere Kenngrössen oder Parameter des Fluids haben. Betrachtet man beispielsweise einen Bioreaktor, in welchem in einer Zellbrühe (cell broth) Proteine oder andere biologische Substanzen hergestellt werden, so ist die Viskosität der Zellbrühe von vielen Faktoren, wie beispielsweise der Temperatur, der Zelldichte, oder der Konzentration des oder der Proteine in der Zellbrühe abhängig. Umgekehrt können dann aus der aktuellen Viskosität Rückschlüsse, gegebenenfalls unter Hinzuziehen weiterer Parameter, über Proteinkonzentrationen, z. B. die Immunoglobulin Konzentration oder allgemein die Konzentration extrazellulärer Proteine, gezogen werden. Je nach Prozess können solche Informationen auch als Abbruchkriterium in der Prozessüberwachung dienen, beispielsweise wenn die Konzentration von Toxinen in der Zellbrühe zu hoch wird.

Insbesondere auch für solche biotechnologischen Prozesse ist es wünschenswert, dass die Bestimmung bzw. die Überwachung der Viskosität inline erfolgen kann, also im Prozess selbst. Hierdurch lässt sich nämlich das sehr aufwändige regelmässige Entnehmen einer Probe des Fluids und die anschliessende Offline Bestimmung der Viskosität vermeiden.

Ein weiteres wichtiges Kriterium bei der Bestimmung der Viskosität ist in vielen Anwendungsfällen die Genauigkeit bzw. die Zuverlässigkeit der Messung.

In der EP-A-1 284 415 wird ein Viskosimeter offenbart, welches eine sehr genaue Bestimmung der Viskosität ermöglicht, wobei zusätzlich auch eine Inline Messung der Viskosität möglich ist. Das Viskosimeter gemäss EP-A-1 284 415 umfasst einen elektrischen Drehantrieb mit einem Stator, der eine Statorwicklung aufweist, und einen in dem Fluid rotierbaren Rotor. Im Betriebszustand ist der Rotor berührungslos magnetisch zur Rotation antreibbar und berührungslos magnetisch bezüglich des Stators lagerbar. Dies wird beispielsweise realisiert, indem der elektromagnetische Drehantrieb nach dem Prinzip des lagerlosen Motors ausgestaltet ist. Da der Rotor in dem Fluid rotiert, dessen Viskosität bestimmt werden soll, hängt das für den Antrieb der Rotation benötigte Drehmoment von der Viskosität der Flüssigkeit ab.

Ein wesentlicher Aspekt ist dabei die berührungslose Lagerung und der berührungslose Antrieb des Rotors, denn hierdurch treten keine Verluste in Form von Lagerreibung oder ähnlichem auf. Der Rotor ist mechanisch vollkommen entkoppelt vom Rest des Drehantriebs, insbesondere vom Stator. Daher hängt die für die Rotation des Rotors im Fluid aufzubringende elektrische Antriebsleistung im Wesentlichen nur von der Viskosität des Fluids ab. Wird im Betrieb durch den Rotor keine oder nur eine vernachlässigbar kleine hydraulische Leistung (Pumpleistung) erbracht, so ist der für die Drehmomentbildung benötigte Strom nur noch von der inneren Reibung und damit von der Viskosität des Fluids abhängig. Somit kann aus dem elektrischen Strom, der zum Generieren des Drehmoments benötigt wird, welches die Rotation des Rotors antreibt, und der Rotationsgeschwindigkeit (Drehfrequenz) des Rotors die Viskosität des Fluids bestimmt werden.

Wenn die hydraulische Leistung des Rotors vernachlässigbar klein ist so ist bei konstanter Drehfrequenz des Rotors die Viskosität des Fluids direkt proportional zum Drehmoment bildenden Strom.

Ist die hydraulische Leistung des Rotors nicht vernachlässigbar, so kann diese beispielsweise durch eine Flussmessung und eine Messung der erzeugten Druckdifferenz bestimmt werden. Die hydraulische Leistung wird dann von der elektrischen Antriebsleistung abgezogen, und aus der verbleibenden Leistung kann dann die Viskosität des Fluids bestimmt werden.

Insbesondere wenn der elektromagnetische Drehantrieb nach dem Prinzip des lagerlosen Motors ausgestaltet ist, erfolgt der Betrieb mittels magnetischer Drehfelder und einer feldorientierten Antriebsregelung (vector control). Bei dieser ist der Drehmoment bildende Strom in einfacher Weise bestimmbar.

Auch wenn sich das in der EP-A-1 284 415 offenbarte Viskosimeter in der Praxis sehr gut bewährt hat, so besteht doch noch Verbesserungsbedarf.

Beispielsweise in der biotechnologischen Industrie wird heute neben einer hohen Messgenauigkeit auch eine sehr schnelle und einfache Anpassung aller Vorrichtungen an den jeweiligen Prozess angestrebt. Dies verlangt dann auch eine möglichst schnelle Austauschbarkeit, sowie eine einfache und möglichst unkomplizierte Handhabung und Bereitstellung der einzelnen Komponenten, selbstverständlich unter der Bedingung, dass keine Zugeständnisse an die Prozesssicherheit oder an die sehr hohen Reinheitsanforderungen vonnöten sind.

Ausgehend von diesem Stand der Technik ist es daher eine Aufgabe der Erfindung, ein Viskosimeter zur Inline Bestimmung der Viskosität eines Fluids vorzuschlagen, dass in sehr einfacher Weise handhabbar ist und zudem eine hohe Messgenauigkeit ermöglicht. Zudem ist es eine Aufgabe der Erfindung, ein Verfahren zur Bestimmung einer Konzentration einer Komponente in einem Fluid vorzuschlagen, welches mit einem solchen Viskosimeter durchführbar ist.

Die diese Aufgaben lösenden Gegenstände der Erfindung sind durch die Merkmale des unabhängigen Patentanspruchs der jeweiligen Kategorie gekennzeichnet.

Erfindungsgemäss wird also ein Viskosimeter zur Inline Bestimmung der Viskosität eines Fluids vorgeschlagen, mit einer Antriebsvorrichtung und mit einer Messvorrichtung, wobei die Messvorrichtung ein Messgehäuse mit einem Einlass und mit einem Auslass für das Fluid umfasst, sowie eine Messkammer, in welcher ein Rotor mit einem ring- oder scheibenförmigen magnetisch wirksamen Kern vorgesehen ist, wobei die Antriebsvorrichtung ein Antriebsgehäuse umfasst, in welchem ein Stator angeordnet ist, welcher mit dem Rotor als elektromagnetischer Drehantrieb zusammenwirkt, wobei der Stator eine Mehrzahl von Spulenkernen aufweist, von denen jeder durch eine Stirnfläche begrenzt wird, und von denen jeder mindestens eine konzentrierte Wicklung trägt, wobei der Stator als Lager- und Antriebsstator ausgestaltet ist, mit welchem der Rotor im Betriebszustand berührungslos magnetisch um eine axiale Richtung antreibbar ist, und mit welchem der Rotor berührungslos magnetisch bezüglich des Stators lagerbar ist, und wobei im Antriebsgehäuse ferner eine Kontrolleinrichtung für die Ansteuerung des Stators und für die Bestimmung der Viskosität anhand einer Betriebsgrösse des elektromagnetischen Drehantriebs vorgesehen ist. Die Messvorrichtung ist zum Einsetzen in die Antriebsvorrichtung ausgestaltet, derart dass die Stirnflächen der Spulenkerne um den magnetisch wirksamen Kern des Rotors herum angeordnet sind, wobei das Messgehäuse lösbar mit dem Antriebsgehäuse verbindbar ist, sodass das Messgehäuse und das Antriebsgehäuse relativ zueinander fixierbar und voneinander trennbar sind.

Ein wesentlicher Aspekt des erfindungsgemässen Viskosimeter ist es, dass das Viskosimeter zwei voneinander separierbare Vorrichtungen umfasst, nämlich eine Messvorrichtung mit einem Messgehäuse und eine Antriebsvorrichtung mit einem Antriebsgehäuse, wobei die Messvorrichtung in einfacher Weise mit der Antriebsvorrichtung verbunden und an dieser fixiert werden kann, nämlich durch ein Einsetzen des Messgehäuses in das Antriebsgehäuse.

Vorzugsweise umfasst die Messvorrichtung alle Komponenten, die während des Messvorgangs mit dem Fluid in Berührung kommen, während die Komponenten der Antriebsvorrichtung nicht mit dem Fluid in Berührung kommen. So ist es beispielsweise möglich, nach Beendigung eines Prozesses die Messvorrichtung von der Antriebsvorrichtung zu trennen, sie zu reinigen und/oder zu sterilisieren, und sie dann wieder in die Antriebsvorrichtung einzusetzen, sodass das Viskosimeter für eine neue Messung oder für einen neuen Prozess bereitsteht. Vorteilhaft ist dabei insbesondere, dass an der Antriebsvorrichtung keine Änderungen und auch keine Reinigungsarbeiten notwendig sind, lediglich die Messvorrichtung muss behandelt und dann wieder in die Antriebsvorrichtung eingesetzt werden.

Dies ermöglicht eine ganz besonders einfache Handhabung des Viskosimeters.

Gemäss einer besonders bevorzugten Ausgestaltung ist die Messvorrichtung als Einmalvorrichtung für den Einmalgebrauch ausgestaltet, und die Antriebsvorrichtung ist als wiederverwendbare Vorrichtung für den Mehrfachgebrauch ausgestaltet. Mit dem Begriff "Einmalvorrichtung" ist dabei gemeint, dass die Vorrichtung für den Einmalgebrauch ausgestaltet ist, also bestimmungsgemäss nur ein einziges Mal benutzt werden kann und dann durch eine neue, noch nicht gebrauchte Einmalvorrichtung ersetzt werden muss. Für eine Messung oder für einen Prozess, wird dann einfach eine noch unbenutzte Messvorrichtung in die Antriebsvorrichtung eingesetzt und an dieser fixiert. Nach Beendigung der Messung oder des Prozesses wird die als Einmalvorrichtung ausgestaltete Messvorrichtung von der Antriebsvorrichtung getrennt und kann dann entsorgt werden.

Gemäss einer bevorzugten Ausführungsform ist der elektromagnetische Drehantrieb, welcher den Rotor und den Stator umfasst, als Tempelmotor ausgestaltet, und jeder Spulenkern umfasst einen stabförmigen Längsschenkel, welcher sich von einem ersten Ende in axialer Richtung bis zu einem zweiten Ende erstreckt, sowie einen Querschenkel, welcher an dem zweiten Ende des Längsschenkels angeordnet ist, und welcher sich in einer radialen Richtung erstreckt, die senkrecht zu der axialen Richtung ist, wobei jeder Querschenkel durch eine der Stirnflächen begrenzt wird, und wobei an jedem Längsschenkel mindestens eine der konzentrierten Wicklungen angeordnet ist, welche den jeweiligen Längsschenkel umgibt. Diese Ausgestaltung als Tempelmotor ist besonders kompakt und platzsparend.

Gemäss einer bevorzugten Ausgestaltung ist das Messgehäuse durch eine Drehung relativ zum Antriebsgehäuse und um die axiale Richtung an dem Antriebsgehäuse fixierbar beziehungsweise von dem Antriebsgehäuse lösbar ist. Die Verbindung und Fixierung zwischen dem Messgehäuse und dem Antriebsgehäuse kann dazu beispielsweise als eine Bajonettverbindung bzw. als ein Bajonettverschluss ausgestaltet sein.

Gemäss einer weiteren bevorzugten Ausgestaltung umfasst die Antriebsvorrichtung eine Verbindungseinrichtung, welche bezüglich des Antriebsgehäuses zwischen einer Offenstellung und einer Schliessstellung drehbar ausgestaltet ist, wobei die Messvorrichtung in die Antriebsvorrichtung einsetzbar und von der Antriebsvorrichtung trennbar ist, wenn sich die Verbindungseinrichtung in der Offenstellung befindet, und wobei das Messgehäuse und das Antriebsgehäuse relativ zueinander fixiert sind, wenn sich die Verbindungseinrichtung in der Schliessstellung befindet. Diese Ausgestaltung hat den Vorteil, dass das Messgehäuse, welches den Einlass und den Auslass für das Fluid umfasst, zum Verbinden mit und zum Trennen von dem Antriebsgehäuse nicht gedreht werden muss. Insbesondere, wenn das Viskosimeter in Fluidsysteme integriert ist, kann es ein Vorteil sein, wenn zum Einsetzen oder zum Entfernen der Messvorrichtung keine Drehung des Einlasses und des Auslasses, insbesondere um die axiale Richtung, notwendig sind.

Vorzugsweise umfasst die Messvorrichtung eine Speichereinheit, in welcher Kalibrierungsdaten für die Messvorrichtung gespeichert sind, wobei die Antriebsvorrichtung eine Schnittstelle umfasst, über welche Daten aus der Speichereinheit an die Kontrolleinrichtung übermittelbar sind. Insbesondere für Messungen, die eine sehr hohe Präzision verlangen, ist es oft notwendig, dass das Viskosimeter kalibriert wird. Hierzu sollte dann üblicherweise die Messvorrichtung gemeinsam mit der Antriebsvorrichtung kalibriert werden. Wird nun die Messvorrichtung, die beispielsweise als Einmalvorrichtung ausgestaltet ist, durch eine neue Messvorrichtung ersetzt, so könnte es notwendig sein, das Viskosimeter, das nun diese neue Messvorrichtung -eingesetzt in die Antriebsvorrichtungumfasst, neu zu kalibrieren. Beispielsweise können geringfügige Variationen in den geometrischen Abmessungen von Komponenten der Messvorrichtung, z. B. geringfügige Variationen im Aussendurchmesser des Rotors oder in seinen magnetischen Eigenschaften, dazu führen, dass nach dem Austausch der Messvorrichtung eine neue Kalibrierung notwendig wird. Solche Variationen sind beispielsweise fertigungstechnisch bedingt und üblicherweise nicht vermeidbar, zumindest nicht mit einem vertretbaren Aufwand.

Um dieses Problem zu lösen, kann beispielsweise jede Messvorrichtung in einem Referenzviskosimeter mit einer Referenz-Antriebsvorrichtung kalibriert werden. Die daraus resultierenden, für die jeweilige Messvorrichtung spezifischen Kalibrierungsdaten werden dann in der Speichereinheit dieser Messvorrichtung gespeichert. Wird nun diese Messvorrichtung in die Antriebsvorrichtung eingesetzt, so werden die spezifischen Kalibrierungsdaten über die Schnittstelle an die Kontrolleinheit der Antriebsvorrichtung übermittelt, sodass eine erneute Kalibrierung des Viskosimeters nach dem Austausch der Messvorrichtung nicht mehr notwendig ist.

Insbesondere für die Ausgestaltung der Messvorrichtung als Einmalvorrichtung ist es bevorzugt, dass die Speichereinheit gammastabil ist, womit gemeint ist, dass die Speichereinheit bei einer Gamma-Sterilisierung keinen Schaden nimmt, sodass insbesondere keine Kalibrierungsdaten verloren gehen. Denn es ist vorteilhaft, wenn die Einmalvorrichtung, also beispielsweise die Messvorrichtung, gamma-sterilisierbar ist. Bei dieser Art der Sterilisierung wird das zu sterilisierende Element mit GammaStrahlung beaufschlagt.

Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen oder Einmalvorrichtungen ist es eine gängige Praxis, dass die Teile nach ihrer Herstellung in die Verpackung gebracht werden und dann noch eine Zeit lagern, bevor sie an den Kunden ausgeliefert werden. In solchen Fällen erfolgt die Sterilisierung durch die Verpackung hindurch, was bei einer Dampfsterilisierung oder anderen Verfahren nicht möglich ist.

Ein weiterer Vorteil der Gamma-Sterilisierung im Vergleich zur Dampfsterilisierung besteht darin, dass die Gamma-Sterilisierung nicht bei einer hohen bzw. erhöhten Temperatur durchgeführt werden muss. Die Dampfsterilisierung hingegen muss bei einer hohen Temperatur durchgeführt werden. Durch die hohe Temperatur besteht die Gefahr, dass insbesondere Kunststoffkomponenten der Einmalvorrichtung zerstört oder beschädigt werden können. Beispielsweise können sich Kunststoffkomponenten verziehen, wodurch wichtige oder kritische Dimensionen der Komponenten verändert werden können.

Als in diesem Sinne gammastabile Speichereinheit können beispielsweise verwendet werden: FRAM (Ferroelectric Random Access Memory), RFID Elemente (RFID: Radio Frequency Identification), oder optoelektronisch lesbare Elemente wie Barcodes oder zweidimensionale Codes, z.B. QR Codes (QR: Quick Response).

Ferner ist es möglich, auch für die Antriebsvorrichtung Kalibrierungsdaten zu ermitteln, die beispielsweise mittels einer Kalibrierung mit einer Referenz-Messvorrichtung bestimmt werden. Die Kalibrierungsdaten für die Antriebsvorrichtung werden dann vorzugsweise in der Kontrolleinrichtung der jeweiligen Antriebsvorrichtung hinterlegt bzw. gespeichert.

Gemäss einer besonders bevorzugten Ausgestaltung umfasst der magnetisch wirksame Kern des Rotors einen Permanentmagneten zur Erzeugung eines Rotormagnetfelds, wobei die Antriebsvorrichtung mindestens einen Magnetfeldsensor umfasst, mit welchem das Rotormagnetfeld ermittelbar ist.

Das von dem Permanentmagneten erzeugte Rotormagnetfeld wird vom magnetisch wirksamen Kern des Rotors durch den Luftspalt zwischen Rotor und Stator hauptsächlich durch die Stirnflächen der Spulenkerne in die Spulenkerne geführt, durch die Spulenkerne hindurch, und dann wieder zurück in den magnetisch wirksamen Kern des Rotors. Wenn es nun zu Änderungen in dem Rotormagnetfeld kommt, so kann dies auch zu Änderungen in dem Drehmoment führen, welches die Rotation des Rotors antreibt. Da dieses Drehmoment aber die Grundlage für die Bestimmung der Viskosität bildet, können Änderungen im Rotormagnetfeld auch zu Änderungen in der Viskositätsmessung führen. Solche Änderungen im Rotormagnetfeld können beispielsweise temperaturbedingt sein. Falls z.B. das Fluid, welches die Messvorrichtung durchströmt, die Temperatur des Rotors erhöht, so resultiert daraus eine Erniedrigung des Rotormagnetfelds, genauer gesagt eine Abnahme der Feldstärke des Rotormagnetfelds.

Mit dem mindestens einen Magnetfeldsensor in der Antriebsvorrichtung kann die aktuelle Stärke des Rotormagnetfelds bestimmt werden. Kommt es nun zu Änderungen im Rotormagnetfeld, so kann dies mittels des Magnetfeldsensors ermittelt werden und es kann eine entsprechende Korrektur vorgenommen werden, sodass die Bestimmung der Viskosität durch die Änderung des Rotormagnetfelds nicht verfälscht wird. Die hierfür notwendigen Korrekturwerte können in der Kontrolleinrichtung hinterlegt bzw. gespeichert sein, beispielsweise in Form einer Lookup-Tabelle oder in Form einer Polynomfunktion.

Besonders bevorzugt ist mindestens ein radialer Magnetfeldsensor vorgesehen, mit welchem eine radiale Komponente des Rotormagnetfelds ermittelbar ist, und zudem vorzugsweise mindestens ein axialer Magnetfeldsensor, mit welchem eine axiale Komponente des Rotormagnetfelds ermittelbar ist.

Da der magnetische Fluss im Luftspalt zwischen dem Rotor und dem Stator hauptsächlich in radialer Richtung verläuft, sind radiale Magnetfeldsensoren besonders geeignet, um auch kleinere Änderungen im Rotormagnetfeld zu detektieren.

Der axiale Magnetfeldsensor ist insbesondere dann vorteilhaft, wenn die Position des Rotors bezüglich der axialen Richtung passiv magnetisch stabilisiert ist, was eine besonders bevorzugte Ausführungsform ist. Dann kann nämlich die Asymmetrie in der Umströmung des Rotors durch das Fluid dazu führen, dass sich die Position des Rotors bezüglich der axialen Richtung ändert. Üblicherweise ist es so, dass das Fluid hauptsächlich über die Oberseite des Rotors strömt und nur ein deutlich geringerer Teil des Fluids unterhalb des Rotors strömt, woraus die genannte Asymmetrie resultiert. Ändert sich nun an der Strömung des Fluids durch die Messvorrichtung etwas, beispielsweise durch eine Zunahme des Durchflusses, so kann dies zu einer Verschiebung des Rotors in axialer Richtung führen. Eine solche axiale Verschiebung führt in der Regel zu einer Änderung des Rotormagnetfelds, was dann wiederum zu einer Änderung in der Viskositätsmessung führen kann. Mit dem axialen Magnetfeldsensor kann eine solche Verschiebung des Rotors in axialer Richtung detektiert werden, und insbesondere kann auch die Richtung der Verschiebung, also nach oben oder nach unten, ermittelt werden. Folglich können Änderungen in der axialen Position des Rotors detektiert und bei der Bestimmung der Viskosität berücksichtigt werden.

Falls mehrere radiale Magnetfeldsensoren und mindestens ein axialer Magnetfeldsensor vorgesehen sind, so ist es auch möglich, den axialen Magnetfeldsensor nur für die Bestimmung des Vorzeichens der axialen Verschiebung zu bestimmen, also ob der Rotor bezüglich der axialen Richtung nach oben oder nach unten verschoben ist. Die Amplitude der axialen Verschiebung, also der Betrag, um welchen der Rotor bezüglich der axialen Richtung verschoben ist lässt sich aus den Signalen der radialen Magnetfeldsensoren bestimmen. Die Amplitude der axialen Verschiebung mithilfe der radialen Magnetfeldsensoren zu bestimmen, hat den Vorteil, dass insbesondere bei mehreren symmetrisch bzw. paarweise symmetrisch angeordneten radialen Magnetfeldsensoren der Einfluss von Verkippungen des Rotors oder der Einfluss von anderen Feldstörungen des magnetischen Feldes kompensiert wird, wodurch die Genauigkeit der Bestimmung der axialen Position des Rotors erhöht werden.

Es ist eine weitere bevorzugte Massnahme, dass die Messvorrichtung einen Temperatursensor umfasst, mit welchem die Temperatur des Fluids in der Messvorrichtung ermittelbar ist. Da für sehr viele Fluide die Viskosität signifikant von der Temperatur abhängt, ist es vorteilhaft, wenn die Temperatur des Fluids dort ermittelt wird, wo auch die Bestimmung der Viskosität erfolgt, nämlich in der Messvorrichtung. Dies ist in vielen Anwendungsfällen genauer als eine Bestimmung der Temperatur des Fluids ausserhalb der Messvorrichtung.

Gemäss einer besonders bevorzugten Ausführungsform ist die Messkammer, in welcher der Rotor angeordnet ist, als eine Ausstülpung in einem Boden des Messgehäuses ausgestaltet, wobei bezüglich der normalen Gebrauchslage in axialer Richtung oberhalb der Messkammer zwischen dem Einlass und dem Auslass eine Hauptströmungsverbindung für das Fluid vorgesehen ist, durch welche das Fluid vom Einlass zum Auslass strömen kann.

Dabei sind solche Ausgestaltungen bevorzugt, bei denen in der Hauptströmungsverbindung ein Flussleitelement vorgesehen ist, welches zentral über dem Rotor angeordnet ist, und welches zum Aufteilen des Fluids in einen ersten Teilfluss und in einen zweiten Teilfluss ausgestaltet ist, derart dass das Flussleitelement zwischen dem Einlass und dem Auslass auf einer Seite von dem ersten Teilfluss umströmt wird, und auf der anderen Seite von dem zweiten Teilfluss umströmt wird. Durch das Flussleitelement, welches einen zentralen Bereich des Rotors überdeckt, wird das Fluid im Betriebszustand in die beiden Teilflüsse aufgeteilt, welche nur noch über die Peripherie der Oberfläche des Rotors strömen. Somit werden mittels des Flussleitelements Rotationsströmungen in der Hauptströmungsverbindung verhindert oder zumindest drastisch reduziert. Solche Rotationsströmungen können sich negativ auswirken, weil sie an der Oberfläche des Rotors auf der einen Seite der Hauptströmung entgegen fliessen und auf der anderen Seite in der gleichen Richtung strömen wie die Hauptströmung. Hierdurch können asymmetrische Reibungseffekte entstehen, welche sich negativ auf die Messung der Viskosität auswirken können.

Ein weiterer Vorteil der Ausgestaltung mit dem Flussleitelement besteht darin, dass dieses einen signifikanten Raum in der Hauptströmungsverbindung in Anspruch nimmt. Dies hat den Vorteil, dass das nasse Volumen, womit der Bereich der Messvorrichtung gemeint ist, welcher mit dem Fluid gefüllt ist, bzw. von dem Fluid durchströmt wird, reduziert wird. Dies ist insbesondere dann ein wichtiger Aspekt, wenn das Fluid ein sehr wertvolles oder teures Fluid ist.

Eine weitere bevorzugte Massnahme besteht darin, dass das Flussleitelement eine Mehrzahl von Seitenkanälen aufweist, welche einen Teil des Fluids aus der Hauptströmungsverbindung in die axiale Richtung auf den Rotor hin umlenken. Durch diese Massnahme lässt sich der Sekundärstrom verstärken, welches der Strom des Fluids ist, der den Rotor umströmt und somit einen wesentlicher Faktor für die Bestimmung der Viskosität darstellt.

Wie bereits erwähnt ist es eine besonders bevorzugte Ausführungsform, dass die Antriebsvorrichtung als wiederverwendbare Vorrichtung für den Mehrfachgebrauch ausgestaltet ist, und die Messvorrichtung als Einmalvorrichtung für den Einmalgebrauch ausgestaltet ist.

Durch die Erfindung wird ferner eine Einmalvorrichtung für den Einmalgebrauch vorgeschlagen, wobei die Einmalvorrichtung als Messvorrichtung für ein erfindungsgemässes Viskosimeter ausgestaltet ist.

Ferner wird durch die Erfindung ein Verfahren zur Bestimmung einer Konzentration einer Komponente in einem Fluid vorgeschlagen, das durch die folgenden Schritte gekennzeichnet ist:
- Bereitstellen eines Viskosimeters, welches gemäss der Erfindung ausgestaltet ist,
- Bereitstellen eines Zusammenhangs zwischen der Konzentration der Komponente in dem Fluid und der Viskosität des Fluids,
- Bestimmen der Viskosität des Fluids mittels des Viskosimeters,
- Bestimmen der Konzentration aus dem Zusammenhang zwischen der Konzentration und der Viskosität.

Da das erfindungsgemässe Viskosimeter eine sehr einfache, präzise und leicht zu handhabende Bestimmung der Viskosität ermöglicht, ist es insbesondere auch dazu geeignet, charakteristische Grössen, Eigenschaften oder Zustände eines Fluids zu bestimmen, die sich mit Hilfe der Viskosität des Fluids bestimmen lassen. Eine wichtige dieser Grössen ist die Konzentration einer Komponente in einem Fluid. Solche Konzentrationsbestimmungen spielen insbesondere auch in der Biotechnologie und in der pharmazeutischen Industrie eine wichtige Rolle. Als Beispiele seien hier die Konzentrationen von Proteinen beispielsweise in einem Bioreaktor oder in eine Zellbrühe genannt. Eine wichtige Anwendung ist beispielsweise die Bestimmung der Immunoglobulin (Antikörper) Konzentration in einem Fluid. Speziell die Bestimmung der Konzentration der Immunoglobuline G (IgG) spielt in der modernen Diagnostik, in der Biotechnologie und bei der Entwicklung von Impfstoffen und Medikamenten eine bedeutende Rolle.

Gemäss einer bevorzugten Ausführung des erfindungsgemässen Verfahrens wird eine Proteinkonzentration in einer Zellbrühe ermittelt, wobei mit dem Viskosimeter eine aktuelle Viskosität der Zellbrühe ermittelt wird, wobei, vorzugsweise photometrisch, eine aktuelle Zelldichte in der Zellbrühe ermittelt wird, wobei anhand eines Referenzwertes für den Zusammenhang zwischen der Zelldichte und der Viskosität ein Korrekturwert für die Viskosität ermittelt wird, wobei aus der aktuellen Viskosität und dem Korrekturwert eine korrigierte Viskosität ermittelt wird, und wobei die Proteinkonzentration in der Zellbrühe aus der korrigierten Viskosität und dem Zusammenhang zwischen der Konzentration und der Viskosität bestimmt wird.

Weitere vorteilhafte Massnahmen und Ausgestaltungen der Erfindung ergeben sich aus den abhängigen Ansprüchen.

Im Folgenden wird die Erfindung sowohl in apparativer als auch in verfahrenstechnischer Sicht anhand von Ausführungsbeispielen und anhand der Zeichnung näher erläutert. In der teilweise schematischen Zeichnung zeigen:
- Fig. 1:: ein erstes Ausführungsbeispiel eines erfindungsgemässen Viskosimeters in einer perspektivischen Ansicht,
- Fig. 2:: wie Fig. 1, jedoch während des Zusammensetzens des Viskosimeters,
- Fig. 3:: wie Fig. 1, jedoch nach dem Zusammensetzen des Viskosimeters,
- Fig. 4:: eine perspektivische Schnittdarstellung des ersten Ausführungsbeispiels,
- Fig. 5:: eine schematische Schnittdarstellung des ersten Ausführungsbeispiels in einem Schnitt entlang der axialen Richtung,
- Fig. 6:: eine perspektivische Schnittdarstellung des elektromagnetischen Drehantriebs des ersten Ausführungsbeispiels,
- Fig. 7-9:: wie Fig. 1-3, jedoch für eine erste Variante des ersten Ausführungsbeispiels,
- Fig. 10:: wie Fig. 5, jedoch für eine zweite Variante des ersten Ausführungsbeispiels,
- Fig. 11:: Wie Fig. 6, jedoch für ein zweites Ausführungsbeispiel eines erfindungsgemässen Viskosimeters,
- Fig. 12:: eine mögliche Ausführungsform für eine Signalverbindung, welche eine elektrische Verbindung zwischen der Messvorrichtung und der Antriebsvorrichtung ermöglicht,
- Fig. 13:: eine Detaildarstellung zur Veranschaulichung der Anordnung eines Temperatursensors,
- Fig. 14:: wie Fig. 13, jedoch für eine andere Ausführungsform,
- Fig. 15:: eine weitere mögliche Ausführungsform für eine Signalverbindung, welche eine elektrische Verbindung zwischen der Messvorrichtung und der Antriebsvorrichtung ermöglicht,
- Fig. 16:: eine Ausführungsform für die Kommunikation zwischen der Speichereinheit der Messvorrichtung und der Antriebsvorrichtung,
- Fig. 17:: die beiden Komponenten für die Kommunikation gemäss der Ausführungsform in Fig. 16,
- Fig. 18:: eine weitere Ausführungsform für die Kommunikation zwischen der Speichereinheit der Messvorrichtung und der Antriebsvorrichtung,
- Fig. 19:: eine Kennzeichnung für die Ausführungsform gemäss Fig. 18,
- Fig. 20:: eine schematische Schnittdarstellung einer Variante für die Messvorrichtung,
- Fig. 21:: eine Aufsicht auf die Variante aus Fig. 20,
- Fig. 22:: eine Schnittdarstellung der Variante aus Fig. 20,
- Fig. 23:: eine Explosionsdarstellung der Variante aus Fig. 20,
- Fig. 24:: ein Diagramm, das den Zusammenhang zwischen der Viskosität und der Konzentration einer Komponente in einem Fluid zeigt,
- Fig. 25:: ein Diagramm, das den Zusammenhang zwischen dem Antriebsstrom bzw. dem Drehmoment und der Konzentration einer Komponente in einem Fluid zeigt,
- Fig. 26:: ein Diagramm, das den Zusammenhang zwischen der Konzentration eines Immunoglobulins und der Viskosität für verschiedene Temperaturen zeigt,
- Fig. 27:: eine schematische Darstellung eines ersten Ausführungsbeispiels eines erfindungsgemässen Verfahrens, bei welchem eine Proteinkonzentration bestimmt wird,
- Fig. 28:: ein Diagramm, das den Zusammenhang zwischen der Zelldichte in einer Zellsuspension und der Viskosität der Zellsuspension zeigt, und
- Fig. 29:: eine schematische Darstellung eines zweiten Ausführungsbeispiels eines erfindungsgemässen Verfahrens.

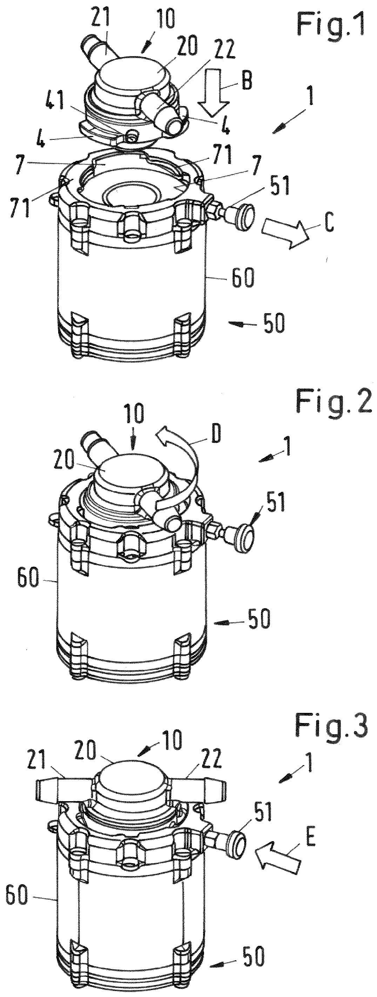

Fig. 1 zeigt eine perspektivische Darstellung eines ersten Ausführungsbeispiel eines erfindungsgemässen Viskosimeters, das gesamthaft mit dem Bezugszeichen 1 bezeichnet ist. Das Viskosimeter 1 umfasst eine Messvorrichtung 10 mit einem Messgehäuse 20, sowie eine Antriebsvorrichtung 50 mit einem Antriebsgehäuse 60. Wie dies noch genauer beschrieben wird, sind die Messvorrichtung 10 und die Antriebsvorrichtung 50 derart ausgestaltet, dass sie in einfacher Weise zusammengesetzt und voneinander getrennt werden können. Besonders bevorzugt ist dieses Zusammensetzen und Trennen von Hand und insbesondere ohne Werkzeug möglich.

Die Messvorrichtung 10 ist zum Einsetzen in die Antriebsvorrichtung 50 ausgestaltet, wobei das Messgehäuse 20 lösbar mit dem Antriebsgehäuse 60 verbindbar ist, sodass das Messgehäuse 20 und das Antriebsgehäuse 60 relativ zueinander fixierbar und voneinander trennbar sind. Das Zusammensetzten von der Messvorrichtung 10 und der Antriebsvorrichtung 50 wird später noch anhand der Fig. 2 und der Fig. 3 erläutert.

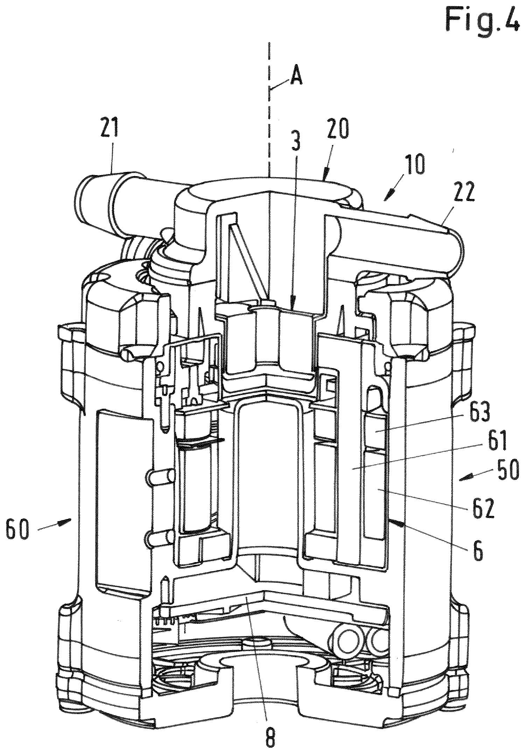

Fig. 4 zeigt eine perspektivische Schnittdarstellung des ersten Ausführungsbeispiels des Viskosimeters 1, wobei ein Viertel des Viskosimeters 1 herausgeschnitten ist.

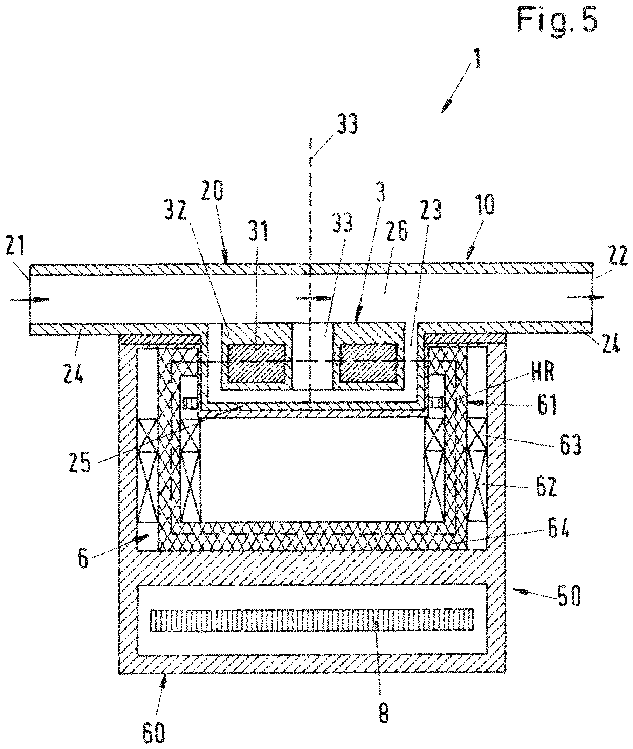

Zum besseren Verständnis zeigt Fig. 5 noch in einer schematischen Darstellung einen Schnitt durch das Viskosimeter 1 in einem Schnitt entlang einer axialen Richtung A.

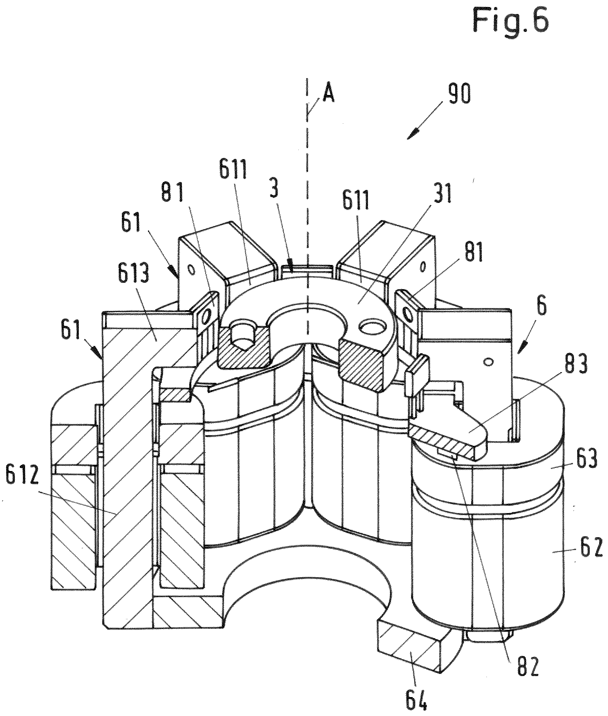

Das erfindungsgemässe Viskosimeter 1 basiert darauf, die Viskosität eines Fluids anhand einer Betriebsgrösse eines elektromagnetischen Drehantriebs 90 zu bestimmen. Fig. 6 zeigt in einer perspektivischen Schnittdarstellung den elektromagnetischen Drehantrieb 90 des ersten Ausführungsbeispiels des Viskosimeters 1.

Das Messgehäuse 20 der Messvorrichtung 10 weist einen Einlass 21 und einen Auslass 22 für das Fluid auf, sowie eine Messkammer 23 (Fig. 5), in welcher ein Rotor 3 mit einem ring- oder scheibenförmigen magnetisch wirksamen Kern 31 vorgesehen ist. Der magnetisch wirksame Kern 31 ist permanentmagnetisch ausgestaltet. Dazu kann der magnetisch wirksame Kern 31 mindestens einen Permanentmagneten, aber auch mehrere Permanentmagnete umfassen oder - wie im hier beschriebenen Ausführungsbeispiel - vollständig aus einem permanentmagnetischen Material bestehen, sodass der magnetisch wirksame Kern 31 der Permanentmagnet ist. Der Permanentmagnet, welcher den magnetisch wirksamen Kern 31 bildet, ist ringförmig ausgestaltet und in diametraler Richtung, also senkrecht zur axialen Richtung A magnetisiert. Der Permanentmagnet erzeugt ein Rotormagnetfeld, welches in Fig. 5 durch die strichlierte Linie mit dem Bezugszeichen HR veranschaulicht ist.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff bzw. ein Material verstanden, der eine Koerzitivfeldstärke, genauer gesagt eine Koerzitivfeldstärke der magnetischen Polarisation aufweist, die mehr als 10'000 A/m beträgt.

Die Messkammer 23, in welcher der Rotor 3 angeordnet ist, ist als eine Ausstülpung 25 in einem Boden 24 des Messgehäuses 20 ausgestaltet. Bezüglich der normalen Gebrauchslage, die in den Fig. 1 bis

Fig. 6 dargestellt ist, ist in axialer Richtung A oberhalb der Messkammer 23 zwischen dem Einlass 21 und dem Auslass 22 eine Hauptströmungsverbindung 26 vorgesehen, durch welche der Hauptfluss des Fluids vom Einlass 21 zum Auslass 22 strömen kann, wie dies die Pfeile ohne Bezugszeichen in Fig. 5 andeuten. Bezüglich der axialen Richtung A ist die Messkammer unterhalb der Hauptströmungsverbindung 26 angeordnet, sodass im Betriebszustand der Rotor 3 von dem Hauptstrom des Fluids überströmt wird.

In dem Antriebsgehäuse 60 der Antriebsvorrichtung 50 ist ein Stator 6 angeordnet, welcher mit dem Rotor 3 als der elektromagnetischer Drehantrieb 90 zusammenwirkt, wobei der Stator 6 eine Mehrzahl von Spulenkernen 61aufweist, von denen jeder durch eine Stirnfläche 611 (siehe Fig. 6) begrenzt wird, und von denen jeder mindestens eine konzentrierte Wicklung 62, 63 trägt.

Im Antriebsgehäuse 60 ist ferner eine Kontrolleinrichtung 8 für die Ansteuerung des Stators 6 und für die Bestimmung der Viskosität anhand einer Betriebsgrösse des elektromagnetischen Drehantriebs 90 vorgesehen. Die Kontrolleinrichtung 8 ist am darstellungsgemäss (Fig. 5) unteren Ende des Antriebsgehäuses 60 im Antriebsgehäuse 60 angeordnet.

Die Antriebsvorrichtung 50 und die Messvorrichtung 10 mit dem in der Messkammer 23 angeordneten Rotor 3 sind so ausgestaltet, dass die Stirnflächen 611 der Spulenkerne 61 um den magnetisch wirksamen Kern 31 des Rotors 3 herum angeordnet sind, wenn die Messvorrichtung 10 in die Antriebsvorrichtung 50 eingesetzt ist. Die Ausstülpung 25 und das Antriebsgehäuse 60 sind also so bemessen, dass die Ausstülpung 25 in eine Ausnehmung des Antriebsgehäuses 60 eingesetzt werden kann, derart, dass die Stirnflächen 611 der Spulenkerne 61 um den magnetisch wirksamen Kerne 31 des Rotors 3 herum angeordnet sind.

Im Folgenden wird nun zunächst der elektromagnetische Drehantrieb 90 näher erläutert.

Der elektromagnetische Drehantrieb 90 (siehe Fig. 6) ist als Tempelmotor ausgestaltet und umfasst den Stator 6, welcher die Mehrzahl von Spulenkernen 61 - hier sechs Spulenkerne 61 - aufweist, von denen jeder einen Längsschenkel 612 umfasst, welcher sich in axialer Richtung A erstreckt, sowie einen senkrecht zum Längsschenkel 612 angeordneten Querschenkel 613, welcher sich in einer radialen Richtung erstreckt, und durch die Stirnfläche 611 begrenzt wird. Die Spulenkerne 61 sind äquidistant auf einer Kreislinie angeordnet, sodass die Stirnflächen 611 den Rotor 3 des elektromagnetischen Drehantriebs 90 umgeben, wenn die Messvorrichtung 10 in die Antriebsvorrichtung 50 eingesetzt ist. An jedem Längsschenkel 612 sind jeweils zwei konzentrierte Wicklungen 62, 63 angeordnet, welche den jeweiligen Längsschenkel 612 umgeben, nämlich eine Antriebsspule 62 und eine Steuerspule 63.

Der Rotor 3 ist bezüglich des Stators 6 berührungslos magnetisch gelagert. Ferner ist der Rotor 3 mittels des Stators 6 berührungslos magnetisch zur Rotation um eine Solldrehachse antreibbar. Die Solldrehachse bezeichnet dabei diejenige Achse, um welche sich der Rotor 3 im Betriebszustand dreht, wenn sich der Rotor 3 bezüglich des Stators 6 in einer zentrierten und unverkippten Lage befindet, so wie dies beispielsweise in Fig. 6 dargestellt ist. Diese Solldrehachse definiert die axiale Richtung A. Üblicherweise stimmt die die axiale Richtung A festlegende Solldrehachse mit der Mittelachse des Stators 6 überein.

Mit einer radialen Richtung wird im Rahmen dieser Anmeldung eine Richtung bezeichnet, welche senkrecht auf der axialen Richtung A steht.

Der Rotor 3 umfasst den magnetisch wirksamen Kern 31, welcher hier ringförmig ausgestaltet ist. Der magnetisch wirksame Kern 31 definiert eine magnetische Mittelebene. Mit der magnetischen Mittelebene des magnetisch wirksamen Kerns 31 des Rotors 3 wird diejenige Ebene senkrecht zur axialen Richtung A bezeichnet, in welcher der magnetisch wirksame Kern 31 des Rotors 3 im Betriebszustand gelagert wird, wenn der Rotor 3 nicht verkippt und in axialer Richtung A nicht ausgelenkt ist. In der Regel ist bei einem scheibenförmigen oder ringförmigen magnetisch wirksamen Kern 31 die magnetische Mittelebene die geometrische Mittelebene des magnetisch wirksamen Kerns 31 des Rotors 3, die senkrecht zur axialen Richtung A liegt. Diejenige Ebene, in welcher der magnetisch wirksame Kern 31 des Rotors 3 im Betriebszustand zwischen den Stirnflächen 611 im Stator 6 gelagert ist, wird auch als radiale Ebene bezeichnet. Die radiale Ebene definiert die x-y-Ebene eines kartesischen Koordinatensystems, dessen z-Achse in axialer Richtung A verläuft. Ist der magnetisch wirksame Kern 31 des Rotors 3 nicht verkippt und bezüglich der axialen Richtung A nicht ausgelenkt, so stimmt die radiale Ebene mit der magnetischen Mittelebene überein.

Mit der radialen Position des magnetisch wirksamen Kerns 31 bzw. des Rotors 3 wird die Lage des Rotors 3 in der radialen Ebene bezeichnet.

In Fig. 6 ist von dem Rotor 3 nur der magnetisch wirksame Kern 31 dargestellt. Es versteht sich, dass der Rotor 3 natürlich auch noch weitere Komponenten umfassen kann wie beispielsweise eine Ummantelung 32 (Fig. 5) oder Kapselung 32, die vorzugsweise aus einem Kunststoff hergestellt sind, oder aus einem Metall oder aus einer Metalllegierung oder aus einer Keramik bzw. einem keramischen Werkstoff.

Wie dies insbesondere in Fig. 5 zu erkennen ist, ist der Rotor 3 gesamthaft in Form einer kreiszylindrischen Ringscheibe vorzugsweise mit einer zentralen Öffnung 33 ausgestaltet, welche sich in axialer Richtung A vollständig durch den Rotor 3 hindurch erstreckt, sodass das Fluid auch durch diese zentrale Öffnung 33 strömen kann.

Der Rotor 3 und insbesondere der magnetisch wirksame Kern 31 des Rotors 3 sind von den radial aussenliegend angeordneten Querschenkeln 613 der Spulenkerne 61 des Stators 6 umgeben, sodass der magnetisch wirksame Kern 31 von den ihm zugewandten Stirnflächen 611 umgeben ist. Die Längsschenkel 612 der Spulenkerne 61 erstrecken sich jeweils von einem ersten Ende, welches das darstellungsgemäss (Fig. 6) untere Ende ist, in axialer Richtung A bis zu einem zweiten Ende, welches das darstellungsgemäss obere Ende ist. Die Querschenkel 613 sind an den oberen Enden der Längsschenkel 612 angeordnet. Jeder Querschenkel 613 erstreckt sich in der radialen Richtung auf den Rotor 3 zu.

Wenn sich der magnetisch wirksame Kern 31 des Rotors 3 während des Betriebs in seiner Soll-Position befindet, so ist der magnetisch wirksame Kern 31 zwischen den Stirnflächen 611 der Querschenkel 613 zentriert, sodass die Querschenkel 613 in der magnetischen Mittelebene bzw. in der radialen Ebene (diese zwei Ebenen sind in diesem Fall gleich) angeordnet sind. Die konzentrierten Wicklungen 62, 63 sind darstellungsgemäss unterhalb der radialen Ebene angeordnet und so ausgerichtet, dass ihre Spulenachsen in axialer Richtung A verlaufen.

Alle ersten Enden der Längsschenkel 612 - also die darstellungsgemäss (Fig. 6) unteren Enden - sind durch einen Rückschluss 64 miteinander verbunden. Der Rückschluss 64 ist vorzugsweise ringförmig ausgestaltet. Dabei sind solche Ausgestaltungen möglich (siehe Fig. 6), bei welchen sich der Rückschluss 64 radial innenliegend entlang aller ersten Enden der Längsschenkel 612 erstreckt. Es ist aber auch möglich, dass der Rückschluss 64 entlang seines Umfangs mehrere Ausnehmungen aufweist, von denen jede eines der ersten Enden aufnimmt. In anderen Ausführungsformen kann der Rückschluss auch eine Mehrzahl von Ringsegmenten umfassen, von denen jedes jeweils zwischen zwei in Umfangsrichtung benachbarten Spulenkernen 61 im Bereich der ersten Enden angeordnet ist. Gemäss anderer Ausführungsformen ist es ferner möglich, den ringförmigen Rückschluss 64 an den ersten Enden der Längsschenkel 612 anzusetzen, derart, dass alle ersten Enden der Längsschenkel 612 auf dem Rückschluss 64 angeordnet sind. Die Längsschenkel 612 sowie der ringförmige Rückschluss 64 sind in diesem Fall in radialer Richtung vorzugsweise gleich dick ausgestaltet. Der Rückschluss 64 besteht dann vorzugsweise aus gewickeltem Blech (Ringbandkern) oder aus einem pulvermagnetischen Werkstoff (auch Soft Magnetic Composites (SMC) genannt).

Um die für den magnetischen Antrieb und die magnetische Lagerung des Rotors 3 notwendigen elektromagnetischen Drehfelder zu erzeugen, tragen die Längsschenkel 612 der Spulenkerne 61 die als konzentrierte Wicklungen ausgestalteten Wicklungen, nämlich die Antriebsspulen 62 und die Steuerspulen 63 wobei bei dem ersten Ausführungsbeispiel um jeden Längsschenkel 612 herum jeweils genau eine Antriebsspule 62 und genau eine Steuerspule 63 angeordnet ist. Mit diesen konzentrierten Wicklungen 62, 63 werden im Betriebszustand diejenigen elektromagnetischen Drehfelder erzeugt, mit welchen ein Drehmoment auf den Rotor 3 bewirkt wird, und mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene, aktiv steuerbar bzw. regelbar ist.

Mit dem "magnetisch wirksamen Kern 31" des Rotors 3 ist derjenige Bereich des Rotors 3 gemeint, welcher für die Drehmomentbildung sowie für die Erzeugung der magnetischen Lagerkräfte magnetisch mit dem Stator 6 zusammenwirkt.

Sowohl der ringförmige Rückschluss 64 als auch die Spulenkerne 61 des Stators 6 sind jeweils aus einem weichmagnetischen Material gefertigt, weil sie zur Führung des magnetischen Flusses dienen.

Geeignete weichmagnetische Materialien für die Spulenkerne 61 und den Rückschluss 64 sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 6 eine Ausgestaltung als Statorblechpaket bevorzugt, bei welcher die Spulenkerne 61 und der Rückschluss 64, geblecht ausgestaltet sind, das heisst sie bestehen aus mehreren dünnen Blechelementen, die gestapelt sind.

Ferner ist es möglich, dass die Spulenkerne 61 und der Rückschluss 64 aus gepressten und anschliessend gesinterten Körnern der genannten Materialien bestehen. Die metallischen Körner sind dabei vorzugsweise in eine Kunststoffmatrix eingebettet, sodass sie zumindest teilweise gegeneinander isoliert sind, wodurch sich Wirbelstromverluste minimieren lassen. Es sind also für den Stator auch weichmagnetische Verbundwerkstoffe geeignet, welche aus elektrisch isolierten und zusammengepressten Metallpartikeln bestehen. Insbesondere können diese weichmagnetischen Verbundwerkstoffe, die auch als SMC (Soft Magnetic Composites) bezeichnet werden, aus Eisenpulverpartikeln bestehen, die mit einer elektrisch isolierenden Schicht beschichtet sind. Diese SMC werden dann mittels pulvermetallurgischer Verfahren zu der gewünschten Ausgestaltung geformt.

Während des Betriebs des elektromagnetischen Drehantriebs 90 wirkt der magnetisch wirksame Kern 31 des Rotors 3 mit dem Stator 6 nach dem auch in der EP 1 284 415 beschriebenen Prinzip des lagerlosen Motors zusammen, bei welchem der Rotor 3 berührungslos magnetisch antreibbar und berührungslos magnetisch bezüglich des Stators 6 lagerbar ist. Dazu ist der Stator 6 als Lager- und Antriebsstator ausgestaltet, mit welchem der Rotor 3 im Betriebszustand berührungslos magnetisch um die Solldrehachse antreibbar - also in Rotation versetzbar- und bezüglich des Stators 6 berührungslos magnetisch lagerbar ist. Dabei sind drei Freiheitsgrade des Rotors 3, nämlich seine Position in der radialen Ebene und seine Rotation, aktiv regelbar. Bezüglich seiner axialen Auslenkung aus der radialen Ebene in axialer Richtung A ist der magnetisch wirksame Kern 31 des Rotors 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene ist der magnetisch wirksame Kern 31 des Rotors 3 ebenfalls passiv magnetisch stabilisiert. Der Rotor 3 ist also durch das Zusammenwirken des magnetisch wirksamen Kerns 31 mit den Spulenkernen 61 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist bezeichnet auch im Rahmen dieser Anmeldung eine aktive magnetische Lagerung eine solche, die aktiv steuer- bzw. regelbar ist, beispielsweise über die mit den Antriebsspulen 62 und Steuerspulen 63 generierten elektromagnetischen Drehfelder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche den Rotor 3 bei einer Auslenkung aus seiner Soll-Position, also z. B. bei einer Verschiebung oder Auslenkung in axialer Richtung A oder bei einer Verkippung, wieder in seine Soll-Position bringen.

Mit einer Radiallagerung oder einer radialen Lagerung wird eine Lagerung des Rotors 3 bezeichnet, mit welcher die radiale Position des Rotors 3 stabilisiert werden kann, also eine Lagerung, welche den Rotor 3 in der radialen Ebene und damit bezüglich seiner radialen Position lagert.

Mit einer Axiallagerung oder einer axialen Lagerung bzw. mit einer Axialstabilisierung oder einer axialen Stabilisierung wird eine Lagerung bzw. eine Stabilisierung des Rotors 3 bezeichnet, mit welcher zum einen die Position des Rotors 3 bezüglich der axialen Richtung A stabilisiert wird, und mit welcher zum anderen der Rotor 3 gegen Verkippungen stabilisiert ist. Solche Verkippungen stellen zwei Freiheitsgrade dar und bezeichnen Auslenkungen, bei denen die momentane Drehachse des Rotors 3 nicht mehr genau in die axiale Richtung A zeigt, sondern einen von Null verschiedenen Winkel mit der Solldrehachse einschliesst. Bei einer Verkippung liegt also die magnetische Mittelebene nicht mehr in oder parallel zur radialen Ebene, sondern die magnetische Mittelebene schliesst einen von Null verschiedenen Winkel mit der radialen Ebene ein.

Gemäss dem Prinzip des lagerlosen Motors werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Antriebsspulen 62 und den Steuerspulen 63 des Stators 6 erzeugten Drehfelder haben in der Regel eine Polpaarzahl, die sich um eins unterscheidet. Hat also beispielsweise das Antriebsfeld die Polpaarzahl p, so hat das Steuerfeld die Polpaarzahl p+1 oder p-1. Dabei werden mit dem Antriebsfeld auf den magnetisch wirksamen Kern 31 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 31 in der radialen Ebene erzeugen, mit welcher die Position des magnetisch wirksamen Kerns 31 in der radialen Ebene regelbar ist. Es ist also nicht möglich, den elektromagnetischen Fluss, der von den Spulen 62, 63 generiert wird, aufzuteilen in einen (elektro-) magnetischen Fluss, der nur für den Antrieb der Rotation sorgt und einen (elektro-) magnetischen Fluss, der nur die magnetische Lagerung realisiert.

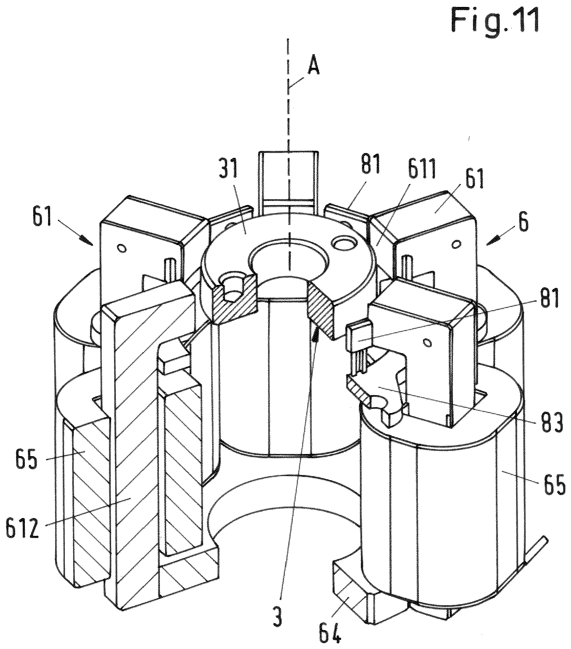

Zur Generierung des Antriebs- und des Steuerfelds ist es einerseits möglich - wie in Fig. 6 gezeigt -, zwei unterschiedliche Wicklungssysteme zu verwenden, nämlich die Antriebsspulen 62 zur Erzeugung des Antriebsfelds und die Steuerspulen 63 zur Erzeugung des Steuerfelds. Der Strom, der in diese Spulen eingeprägt wird, wird dann als Antriebsstrom bzw. als Steuerstrom bezeichnet. Andererseits ist es aber auch möglich (siehe das zweite Ausführungsbeispiel, das in Fig. 11 dargestellt ist), die Antriebs- und Lagerfunktion mit nur einem einzigen Wicklungssystem zu generieren, sodass es also keine Unterscheidung zwischen Antriebs- 62 und Steuerspulen 63 gibt. Dies kann so realisiert werden, dass die von der Kontrolleinrichtung 8 jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 65 (Fig. 11) eingeprägt wird. In diesem Fall ist es natürlich nicht mehr möglich, zwischen Steuer- und Antriebsspulen zu unterscheiden.

In dem hier beschriebenen ersten Ausführungsbeispiel sind im Stator 6 zwei getrennten Wicklungssysteme vorgesehen, nämlich eines mit den separaten Antriebsspulen 62 und eines mit den separaten Steuerspulen 63.

Das erfindungsgemässe Viskosimeter basiert auf dem Prinzip, den Rotor in der Messkammer 23 und damit in dem Fluid zu rotieren und die Viskosität aus der elektrischen Antriebsleistung zu ermitteln, die benötigt wird, um die Rotation des Rotors 3 im Fluid mit einer bekannten Drehzahl anzutreiben.

Das magnetische Antriebsfeld, das mit den Antriebsspulen 62 generiert wird, ist ein elektromagnetisches Drehfeld, welches ein Drehmoment auf den magnetisch wirksamen Kern 31 des Rotors 3 ausübt und dadurch den Rotor 3 in Rotation versetzt. Das Antriebsfeld kann dabei nach dem bekannten Verfahren der feldorientierten Regelung (vector control) über einen Antriebsstrom kontrolliert werden. Im Rahmen dieser Anmeldung bezeichnet der Begriff "Antriebsstrom" denjenigen Strom, der in den Stator 6 des elektromagnetischen Drehantriebs 90 eingespeist werden muss, um das Drehmoment zu generieren, welches die Rotation des Rotors 3 antreibt.

Wird im Betrieb durch den Rotor 3 keine oder nur eine vernachlässigbar kleine hydraulische Leistung (Pumpleistung) erbracht, so ist der für die Drehmomentbildung benötigte Antriebsstrom nur noch von der inneren Reibung und damit von der Viskosität des Fluids abhängig. Somit kann aus dem elektrischen Antriebsstrom, der zum Generieren des Drehmoments benötigt wird, welches die Rotation des Rotors 3 antreibt, und der Rotationsgeschwindigkeit (Drehfrequenz) des Rotors 3 die Viskosität des Fluids bestimmt werden.

Im Folgenden wird davon ausgegangen, dass der Rotor 3 höchstens eine vernachlässigbar kleine hydraulische Leistung (Pumpleistung) erbringt. Falls die hydraulische Leistung nicht vernachlässigbar klein sein sollte, so kann die hydraulische Leistung in einfacherweise messtechnisch bestimmt werden und dann abgezogen werden, sodass nur noch die für die Rotation des Rotors benötigte Antriebsleistung übrigbleibt.

Im Rahmen dieser Anmeldung wird auf den elektrischen Strom als eine mögliche Betriebsgrösse des elektromagnetischen Drehantriebs Bezug genommen. Es versteht sich jedoch, dass der elektrische Strom nur als ein Beispiel einer Betriebsgrösse oder einer elektrischen Betriebsgrösse zu verstehen ist. Die Erläuterungen gelten in sinngemäss gleicher Weise auch für andere Betriebsgrössen wie beispielsweise die Drehzahl, mit welcher der Rotor 3 rotiert, oder für andere elektrische Betriebsgrössen, wie beispielsweise die elektrische Spannung oder die elektrische Leistung.

Im Betriebszustand wird also die Messvorrichtung 10 von dem Fluid durchströmt. Dabei strömt der grösste Teil des Fluids als Hauptstrom durch die Hauptströmungsverbindung 26, während ein kleinerer Sekundärstrom den Rotor 3 umströmt bzw. durch die zentrale Öffnung 33 durchströmt. Der Rotor 3 wird zur Rotation mit einer konstanten und vorgebbaren Rotationsgeschwindigkeit bzw. Drehzahl angetrieben. Der für diese Rotationsgeschwindigkeit bzw. Drehzahl benötigte Antriebsstrom wird ermittelt, und daraus wird dann - beispielsweise in der Kontrolleinrichtung 8 - die Viskosität des Fluids bestimmt.

Ferner ist es auch möglich, die Antriebsvorrichtung 50 bzw. den Stator 6 des elektromagnetischen Antriebs 90 mit einem konstanten, einstellbaren oder vorgebbaren Antriebsstrom zu beaufschlagen, welcher ein konstantes Drehmoment erzeugt. In Abhängigkeit der Viskosität des Fluids stellt sich dann eine Drehzahl ein, welche zur Bestimmung der Viskosität verwendet werden kann. Bei einem vorgegebenen Antriebsstrom beziehungsweise Drehmoment stellt sich ein umgekehrt proportionaler Zusammenhang zwischen der Drehzahl und der Viskosität ein, das heisst, eine geringere Viskosität resultiert in einer höheren Drehzahl bzw. Rotationsgeschwindigkeit des Rotors 3 und eine höhere Viskosität in einer geringeren Drehzahl. Diese Zusammenhänge können für unterschiedliche Antriebsströme als dreidimensionales Kennlinienfeld in der Kontrolleinrichtung 8 abgelegt werden oder durch algebraische Formeln zumindest näherungsweise abgebildet werden. Der Antriebsstrom wird dann im Betrieb so gewählt, dass der zu erwartende Viskositätsbereich durch den Drehzahlbereich der Antriebsvorrichtung 50 abgedeckt werden kann.

Gemäss einer bevorzugten Massnahme umfasst die Antriebsvorrichtung 50 mindestens einen Magnetfeldsensor (Fig. 6), mit welchem das Rotormagnetfeld HR ermittelbar ist. Bei dem ersten Ausführungsbeispiel sind mehrere Magnetfeldsensoren vorgesehen, nämlich eine Mehrzahl von radialen Magnetfeldsensoren 81, von denen jeder so angeordnet ist, dass er an seinem Ort die radiale Komponente des Rotormagnetfeldes (HR) messen kann, und mindestens ein axialer Magnetfeldsensor 82, der so angeordnet ist, dass er an seinem Ort die axiale Komponente des Rotormagnetfelds HR messen kann. Vorzugsweise ist jeder Magnetfeldsensor 81, 82 als Hallsensor ausgestaltet, aber natürlich eignen sich auch alle anderen Arten von Magnetfeldsensoren für das erfindungsgemässe Viskosimeter 1.

Wie dies insbesondere Fig. 6 zeigt, ist jeweils zwischen zwei in Umfangsrichtung benachbarten Querschenkeln 613 einer der radialen Magnetfeldsensoren 81 angeordnet, sodass hier insgesamt sechs radiale Magnetfeldsensoren 81 vorgesehen sind. Bezüglich der axialen Richtung A sind alle radialen Magnetfeldsensoren 81 auf der gleichen Höhe angeordnet wie der magnetisch wirksame Kern 31 des Rotors 3, sodass alle radialen Magnetfeldsensoren 81 dem magnetisch wirksamen Kern 31 gegenüberliegen und diesem zugewandt sind.

Vorzugsweise sind alle radialen Magnetfeldsensoren 81 auf einer Sensor-Platine bzw. auf einem Sensor-PCB (PCB printed circuit board) 83 angeordnet. Das Sensor-PCB 83 ist vorzugsweise im Wesentlichen ringförmig ausgestaltet und bezüglich der axialen Richtung A unterhalb der Querschenkel 613 angeordnet, nämlich in dem Raum zwischen den Steuerspulen 63 und den Querschenkeln 613. Auf dem Sensor-PCB ist ferner vorzugsweise mindestens ein axialer Magnetfeldsensor 82 vorgesehen, welcher so angeordnet und ausgerichtet ist, dass mit ihm die axiale Komponente des Rotormagnetfelds HR an seinem Ort bestimmt werden kann.

Auf dem Sensor-PCB 83 können auch noch weitere Sensoren vorgesehen sein, beispielsweise Positionssensoren für die Bestimmung der Position des Rotors 3 in der radialen Ebene oder andere Sensoren, die für den Betreib des elektromagnetischen Drehantriebs 90 notwendig oder vorteilhaft sind. Das Sensor-PVB 83 ist mit der Kontrolleinrichtung 8 signalverbunden, sodass die mit den Magnetfeldsensoren 81, 82 bestimmten Messwerte an die Kontrolleinrichtung 8 übermittelt werden können.

Durch die Magnetfeldsensoren 81, 82 kann die Bestimmung der Viskosität noch weiter verbessert werden, weil Änderungen im Rotormagnetfeld HR detektiert und dann auch kompensiert werden können, sodass Verfälschungen der Viskositätsbestimmung vermieden werden können.

Solche Änderungen im Rotormagnetfeld HR können beispielsweise durch Temperaturänderungen des Permanentmagneten im magnetisch wirksamen Kern 31 des Rotors 3 verursacht werden. So ist es beispielsweise möglich, dass das durch die Messvorrichtung 10 strömende Fluid den magnetisch wirksamen Kern 31 des Rotors 3 aufheizt, sodass sich dessen Temperatur erhöht. Durch diese Temperaturerhöhung reduziert sich dann das Rotormagnetfeld HR. Derartige Änderungen des Rotormagnetfeldes HR können insbesondere mit den radialen Magnetfeldsensoren 81 mit hoher Genauigkeit und mit grosser Zuverlässigkeit detektiert werden.

Eine weitere Ursache für Änderungen im Rotormagnetfeld HR kann die Asymmetrie des hydraulischen Flusses am Rotor 3 sein. Die Oberseite des Rotor 3 wird wesentlich stärker überströmt, nämlich durch den Hauptstrom in der Hauptströmungsverbindung 26 als andere Bereiche des Rotors 3. Die dem Boden 24 zugewandte Oberfläche des Rotors 3 ist beispielsweise einer deutlich geringeren Strömung ausgesetzt. Kommt es nun beispielsweise zu Änderungen im Gesamtfluss des Fluids durch die Messvorrichtung 10, so ändert sich hierdurch besonders merklich der Hauptstrom oberhalb des Rotors 3. Dies kann dazu führen, dass sich die Position des Rotors 3 bezüglich der axialen Richtung A verändert. Durch die Änderung in der hydraulischen Umströmung kann der Rotor 3 angehoben werden, sich also vom Boden 24 entfernen, oder der Rotor 3 kann abgesenkt werden, also sich näher an den Boden 24 bewegen. Solche Änderungen in der axialen Position des Rotors 3 führen zu Änderungen im Rotormagnetfeld HR. Derartige Änderungen können besonders gut mit dem axialen Magnetfeldsensor 82 detektiert und dann in der Kontrolleinrichtung 8 bei der Bestimmung der Viskosität kompensiert werden.

Falls - wie in Fig. 6 dargestellt - mehrere radiale Magnetfeldsensoren 81 und mindestens ein axialer Magnetfeldsensor 82 vorgesehen sind, so ist es auch möglich, den axialen Magnetfeldsensor 82 nur für die Bestimmung des Vorzeichens der axialen Verschiebung zu bestimmen, also ob der Rotor 3 bezüglich der axialen Richtung A nach oben oder nach unten verschoben ist. Die Amplitude der axialen Verschiebung, also der Betrag, um welchen der Rotor 3 bezüglich der axialen Richtung A verschoben ist, lässt sich aus den Signalen der radialen Magnetfeldsensoren 81 bestimmen. Die Amplitude der axialen Verschiebung mithilfe der radialen Magnetfeldsensoren 81 zu bestimmen, hat den Vorteil, dass insbesondere bei mehreren symmetrisch bzw. paarweise symmetrisch angeordneten radialen Magnetfeldsensoren 81 der Einfluss von Verkippungen des Rotors oder der Einfluss von anderen Feldstörungen des Rotormagnetfelds HR, z.B. durch magnetische Störfelder, kompensiert wird, wodurch die Genauigkeit der Bestimmung der axialen Position des Rotors 3 erhöht werden

Vorzugsweise umfasst die Messvorrichtung 10 alle Komponenten, die während des Messvorgangs mit dem Fluid in Berührung kommen, während die Komponenten der Antriebsvorrichtung 50 nicht mit dem Fluid in Berührung kommen. So ist es beispielsweise möglich, nach Beendigung eines Prozesses die Messvorrichtung 10 von der Antriebsvorrichtung 50 zu trennen, sie zu reinigen und/oder zu sterilisieren, und sie dann wieder in die Antriebsvorrichtung 50 einzusetzen, sodass das Viskosimeter 1 für eine neue Messung oder für einen neuen Prozess bereitsteht. Dies ermöglicht eine ganz besonders einfache Handhabung des Viskosimeters.

Gemäss einer besonders bevorzugten Ausgestaltung ist die Messvorrichtung 10 als Einmalvorrichtung für den Einmalgebrauch ausgestaltet, und die Antriebsvorrichtung 50 ist als wiederverwendbare Vorrichtung für den Mehrfachgebrauch bzw. für den Dauergebrauch ausgestaltet.

Mit dem Begriff "Einmalvorrichtung" und anderen Zusammensetzungen mit dem Bestandteil "Einmal", wie z. B. Einmalteil, Einmalkomponente usw., sind dabei solche Komponenten bzw. Teile gemeint, die für den Einmalgebrauch ausgestaltet sind, die also bestimmungsgemäss nur ein einziges Mal benutzt werden können und dann entsorgt werden. Für eine neue Anwendung muss dann ein neues, bisher unbenutztes Einmalteil eingesetzt werden. Bei der Konzipierung bzw. der Ausgestaltung der der Messvorrichtung 10 als Einmalvorrichtung sind es daher wesentliche Aspekte, dass die Einmalvorrichtung möglichst einfach und wirtschaftlich herstellbar ist, wenige Kosten verursacht und aus möglichst preisgünstig erhältlichen Materialen, beispielsweise Kunststoffen, herstellbar ist. Ein anderer wesentlicher Aspekt ist es, dass die Einmalvorrichtung, hier also die Messvorrichtung 10, in möglichst einfacher Weise mit der wiederverwendbaren Vorrichtung, hier also der Antriebsvorrichtung zu dem Viskosimeter 1 zusammenfügbar ist. Die Einmalvorrichtung soll also in sehr einfacher Weise ersetzt werden können, ohne dass dafür ein hoher Montageaufwand notwendig ist. Besonders bevorzugt soll die Einmalvorrichtung ohne die Verwendung von Werkzeugen mit der wiederverwendbaren Vorrichtung zusammenfügbar bzw. von dieser trennbar sein.

Auch ist es ein wichtiger Aspekt, dass die Einmalvorrichtung nach ihrem Gebrauch möglichst einfach zu entsorgen ist. Daher werden solche Materialien bevorzugt, die eine möglichst geringe Umweltbelastung, insbesondere auch bei der Entsorgung mit sich bringen.

Ferner ist es bei der Ausgestaltung der Messvorrichtung 10 als Einmalvorrichtung besonders bevorzugt, dass die aus Kunststoff gefertigten Teile, also beispielsweise das Messgehäuse 20 oder die Ummantelung 32 aus einem möglichst preisgünstigen, handelsüblichen Kunststoff hergestellt werden. Ein weiterer wesentlicher Aspekt ist es, dass die als Einmalvorrichtung ausgestaltete Messvorrichtung 10 bzw. ihre Komponenten für gewisse Anwendungsbereiche sterilisierbar sein müssen. Dabei ist es besonders vorteilhaft, wenn die als Einmalvorrichtung ausgestaltete Messvorrichtung 10 gamma-sterilisierbar ist. Bei dieser Art der Sterilisierung wird das zu sterilisierende Element mit GammaStrahlung beaufschlagt. Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen ist es eine gängige Praxis, dass die Teile nach ihrer Herstellung in die Verpackung gebracht werden und dann noch eine Zeit lagern, bevor sie an den Kunden ausgeliefert werden. In solchen Fällen erfolgt die Sterilisierung durch die Verpackung hindurch, was bei einer Dampfsterilisierung oder anderen Verfahren nicht möglich ist.

Ein weiterer Vorteil der Gamma-Sterilisierung im Vergleich zur Dampfsterilisierung besteht darin, dass die Gamma-Sterilisierung nicht bei einer hohen bzw. erhöhten Temperatur durchgeführt werden muss. Die Dampfsterilisierung hingegen muss bei einer hohen Temperatur durchgeführt werden. Durch diese hohe Temperatur besteht die Gefahr, dass insbesondere Kunststoffkomponenten der Einmalvorrichtung zerstört oder beschädigt werden können. Beispielsweise können sich Kunststoffkomponenten verziehen, wodurch wichtige oder kritische Dimensionen der Komponenten, beispielsweise der Aussendurchmesser des Rotors 3, verändert werden können.

Die als Einmalvorrichtung ausgestaltete Messvorrichtung 10 bietet durch ihre nur einmalige Verwendbarkeit den grossen Vorteil, dass man bei der Konstruktion keinen Wert auf eine gute Reinigbarkeit der Einmalvorrichtung legen muss, weil die Einmalvorrichtung bei bestimmungsgemässem Gebrauch nicht gereinigt werden muss. Ferner ist es in der Regel nicht notwendig, dass die Einmalvorrichtung bzw. ihre Komponenten mehr als einmal sterilisierbar sein müssen. Dies ist insbesondere bei der Gamma-Sterilisierung ein grosser Vorteil, weil die Beaufschlagung mit GammaStrahlung bei Kunststoffen zu Degradationen führen kann, sodass eine mehrfache Gamma-Sterilisierung den Kunststoff unbrauchbar machen kann.

Da in der Regel bei Einmalteilen auf eine Sterilisierung unter hohen Temperaturen und /oder unter hohem (Dampf-) Druck verzichtet werden kann, können kostengünstigere Kunststoffe eingesetzt werden, beispielsweise solche, die keine hohen Temperaturen aushalten, oder die nicht mehrfach hohen Temperatur- und Druckwerten ausgesetzt werden können.

Unter Berücksichtigung all dieser Aspekte ist es daher bevorzugt, für die Herstellung der Messvorrichtung 10 als Einmalvorrichtung solche Kunststoffe und solche Komponenten zu verwenden, die zumindest einmal gamma-sterilisierbar sind. Die Materialien und die Komponenten sollten dabei gammastabil für eine Dosis von mindestens 40 kGy sein, um eine einmalige Gamma-Sterilisierung zu ermöglichen. Bei der Gamma-Sterilisierung sollten zudem keine giftigen Stoffe entstehen. Zudem ist es bevorzugt, wenn alle Materialien, die mit dem Fluid in Berührung kommen, USP Class VI Standards erfüllen. Ferner ist es bevorzugt, dass alle Materialien, die mit dem Fluid in Berührung kommen, frei sind von Komponenten tierischen Ursprungs (animal free), um Verunreinigungen des Fluids mit Prionen zu verhindern. Prionen könnten nämlich zu gefährlichen Erkrankungen wie BSE oder TSE führen.

Für die Herstellung der aus Kunststoff bestehenden Teile der Messvorrichtung 10 sind beispielsweise folgende Kunststoffe bevorzugt: PolyEthylene (PE), PolyPropylene (PP), Low Density PolyEthylene (LDPE), Ultra Low Density PolyEthylene (ULDPE), High Density PolyEthylene (HDPE), Ethylene Vinyl Acetate (EVA), PolyEthylene Terephthalate (PET), PolyVinylChlorid (PVC), PolyVinyliDene Fluoride (PVDF), Acrylonitrile Butadiene Styrene (ABS), PolyAcryl, PolyCarbonate (PC).

Weniger geeignete oder sogar ungeeignete Materialien für die Herstellung der Kunststoffteile der Messvorrichtung 10 sind beispielsweise die unter dem Markennamen Teflon bekannten Materialien Polytetrafluoroethylene (PTFE) und Perfluoralkoxy-Polymere (PFA). Bei diesen Materialien besteht nämlich bei der Gamma-Sterilisierung die Gefahr, das gefährliche Gase austreten, wie beispielsweise Fluor, das dann giftige oder schädliche Verbindungen wie Flusssäure (HF) bilden kann.

Im Hinblick auf Anwendungen in der Biotechnologie ist es bevorzugt, dass zumindest die Komponenten, die mit dem Fluid in Kontakt kommen aus einem biokompatiblen Material bestehen.

Anhand der Fig. 1 bis Fig. 3 wird nun das einfache Zusammensetzen der Messvorrichtung 10 mit der Antriebsvorrichtung 50 erläutert. Bei dem ersten Ausführungsbeispiel sind die Messvorrichtung 10 und die Antriebsvorrichtung 50 über eine Bajonettverbindung miteinander verbindbar und voneinander lösbar. Dazu umfasst das Messgehäuse 20 mehrere, hier drei, sektorförmige Vorsprünge 4, welche entlang des Umfangs des Messgehäuses 20 angeordnet sind. Im oberen Randbereich des Antriebsgehäuse 60 ist eine Mehrzahl von Ausnehmungen 7 entlang des inneren Umfangs des Antriebsgehäuses 60 angeordnet, wobei die Anzahl der Ausnehmungen 7 der Anzahl der Vorsprünge 4 entspricht. Die Ausnehmungen 7 sind bezüglich der Umfangsrichtung so bemessen und angeordnet, dass jeweils einer der Vorsprünge 4 in axialer Richtung A in eine der Ausnehmungen 7 eingebracht werden kann. Zwischen zwei in Umfangsrichtung benachbarten Ausnehmungen ist jeweils eine Lasche 71 angeordnet, welche in radialer Richtung nach innen gesehen die Ausnehmungen 7 überragt. Jede Lasche 71 ist derart ausgestaltet, dass sie einen der Vorsprünge 4 übergreifen kann. Ferner ist im oberen Randbereich der Antriebsgehäuses 60 ein beispielsweise federbelasteter Sicherungsstift 51 vorgesehen, der in radialer Richtung nach aussen gezogen werden kann. Am Messgehäuse 20 ist eine Sicherungsöffnung 41 vorgesehen, die derart ausgestaltet und angeordnet ist, dass der Sicherungsstift 51 in die Sicherungsöffnung 41 eingreift, falls die Messvorrichtung 10 an der Antriebsvorrichtung 50 fixiert ist.

Zum Herstellen und Fixieren der Verbindung der Messvorrichtung 10 mit der Antriebsvorrichtung 50 wird der Sicherungsstift 51 in radialer Richtung nach aussen gezogen, wie dies der Pfeil C in Fig. 1 zeigt. Die Messvorrichtung 10 wird in axialer Richtung A in die Antriebsvorrichtung 50 eingeführt, sodass jede der Ausnehmungen 7 jeweils einen der Vorsprünge 4 aufnimmt. Dies ist in Fig. 1 durch den Pfeil B angedeutet. Fig. 2 zeigt den Zustand, dass die Vorsprünge 4 vollständig in die Ausnehmungen 7 eingeführt sind. Nun wird die Messvorrichtung 10 um den Bruchteil einer Umdrehung um die axiale Richtung A gedreht (siehe Pfeil D in Fig. 2), sodass sich die Vorsprünge 4 unter die Laschen 71 schieben. Danach ist jeder Vorsprung 4 von einer der Laschen 71 übergriffen, sodass die Messvorrichtung 10 an der Antriebsvorrichtung 50 fixiert ist. Schliesslich wird noch der Sicherungsstift 51 radial nach innen bewegt (siehe Pfeil E in Fig. 3), sodass der Sicherungsstift 51 in die Sicherungsöffnung 41 eingreift und ein weiteres Verdrehen der Messvorrichtung 10 relativ zur Antriebsvorrichtung 50 verhindert. Falls der Sicherungsstift 51 federbelastet ausgestaltet ist, greift er selbsttätig in die Sicherungsöffnung 41 ein, sobald diese in der korrekten Position ist.

Fig. 3 zeigt das Viskosimeter im zusammengesetzten und betriebsbereiten Zustand, mit der in die Antriebsvorrichtung 50 eingesetzten und an dieser fixierten Messvorrichtung 10. Das Trennen der Messvorrichtung 10 von der Antriebsvorrichtung 50 erfolgt in sinngemäss umgekehrter Weise wie das Zusammensetzen.

Es versteht sich, dass die Vorsprünge 4 nicht alle gleich ausgestaltet sein müssen. Sie können sich beispielsweise durch ihre Länge in Umfangsrichtung voneinander unterscheiden. Dies kann vorteilhaft sein, wenn die Messvorrichtung 10 nur in genau einer Orientierung in die Antriebseinrichtung 50 einsetzbar sein soll.

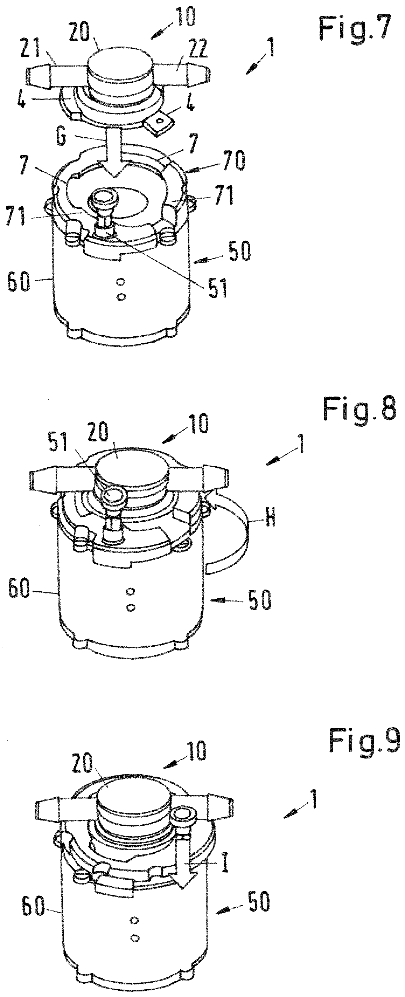

In den Fig. 7 bis Fig. 9 ist in einer den Fig. 1 bis Fig. 3 entsprechenden Darstellung eine erste Variante des ersten Ausführungsbeispiels dargestellt. Diese erste Variante unterscheidet sich nur durch die Ausgestaltung der Verbindung zwischen der Messvorrichtung 10 und der Antriebsvorrichtung 50. Im Folgenden wird nur auf die Unterschiede zwischen der ersten Variante und dem ersten Ausführungsbeispiel eingegangen. Ansonsten gelten die vorangehenden Erläuterungen in sinngemäss gleicher Weise auch für die erste Variante.

Bei der ersten Variante umfasst die Antriebsvorrichtung 50 eine Verbindungseinrichtung 70, welche ringförmig ausgestaltet und am oberen Randbereich des Antriebsgehäuses 60 angeordnet ist, derart, dass die Verbindungseinrichtung 70 den oberen Randbereich des Antriebsgehäuses 60 bildet. Die ringförmige Verbindungseinrichtung 70 ist so ausgestaltet, dass sie bezüglich des Antriebsgehäuses 60 zwischen einer Offenstellung, die in Fig. 7 und in Fig. 8 dargestellt ist, und einer Schliessstellung, die in Fig. 9 dargestellt ist, drehbar ist. Die Ausnehmungen 7 mit den dazwischen angeordneten Laschen 71 sind in bzw. an der Verbindungseinrichtung 70 vorgesehen.

Wie dies insbesondere in Fig. 7 zu erkennen ist, sind bei der ersten Variante die Vorsprünge 4 - und dementsprechend auch die Ausnehmungen 7 -mit unterschiedlicher Länge in Umfangsrichtung ausgestaltet, sodass die Messvorrichtung 10 nur in genau einer Position in die Antriebsvorrichtung 50 eingesetzt werden kann.

Die Messvorrichtung 10 ist nur dann in die Antriebsvorrichtung 50 einsetzbar und von der Antriebsvorrichtung 50 trennbar, wenn sich die Verbindungseinrichtung 70 in der Offenstellung befindet, die in Fig. 7 und in Fig 8 dargestellt ist. Wenn sich die Verbindungseinrichtung 70 in der Schliessstellung befindet, die in Fig. 9 dargestellt ist, übergreifen die Laschen 71, die an der Verbindungseinrichtung 70 vorgesehen sind, die Vorsprünge 4, sodass das Messgehäuse 20 und das Antriebsgehäuse 60 relativ zueinander fixiert sind.

Der Sicherungsstift 51 ist bei der ersten Variante so ausgestaltet, dass er die Verbindungseinrichtung 70 gegen eine Drehung relativ zum Antriebsgehäuse 60 sichert, wenn sich die Verbindungseinrichtung 70 in der Schliessstellung befindet. Dazu ist der Sicherungsstift 51 bei der ersten Variante in axialer Richtung A beweglich ausgestaltet. Wenn sich die Verbindungseinrichtung 70 in der Schliessstellung befindet, so greift der an der Verbindungseinrichtung 70 fixierte Sicherungsstift 51 in eine Sicherungsöffnung im Antriebsgehäuse 60 ein, sodass die Verbindungseinrichtung 70 in der Schliessstellung fixiert ist und nicht mehr relativ zum Antriebsgehäuse 60 gedreht werden kann, ohne vorher den Sicherungsstift 51 in axialer Richtung A zu betätigen.

Zum Zusammensetzen wird die Verbindungseinrichtung 70 in die Offenstellung gebracht, die in Fig. 7 gezeigt ist. Die Messvorrichtung 10 wird dann durch die Verbindungseinrichtung 70 hindurch in die Antriebseinrichtung 50 eingesetzt, wie dies der Pfeil G in Fig. 7 andeutet. Dabei werden die Vorsprünge 4 von den Ausnehmungen 7 aufgenommen. Wenn die Messvorrichtung 10 in die Antriebsvorrichtung eingesetzt ist (siehe Fig. 8) wird die Verbindungseinrichtung 70 durch eine Drehung relativ zum Antriebsgehäuse 60 und um die axiale Richtung A aus der Offenstellung in die Schliessstellung gedreht, wie dies der Pfeil H in Fig. 8 andeutet. Wenn die Verbindungseinrichtung 70 in der in Fig. 9 dargestellten Schliessstellung ist, wird der Sicherungsstift 51 in axialer Richtung A darstellungsgemäss nach unten bewegt, wie dies der Pfeil I in Fig. 9 andeutet, sodass der Sicherungsstift 51 in die Sicherungsöffnung im Antriebsgehäuse 60 eingreift und ein weiteres Verdrehen der Verbindungseinrichtung 70 relativ zum Antriebsgehäuse 60 verhindert. Falls der Sicherungsstift 51 federbelastet ausgestaltet ist, greift er selbsttätig in die Sicherungsöffnung ein, sobald sich die Verbindungseinrichtung 70 in der Schliessstellung befindet.

Die in Fig. 7 bis Fig. 9 dargestellte erste Variante ist insbesondere dann vorteilhaft, wenn das Viskosimeter 1 in ein Fluidsystem integriert ist, in welchem es nicht möglich ist, bei einem Austausch der Messvorrichtung 10 die Messvorrichtung 10 relativ zur Antriebsvorrichtung 50 zu verdrehen, beispielsweise weil der Einlass 21 und/oder der Auslass 22 an starre, d.h. nicht flexibles Leitungen angeschlossen werden muss.

In anderen Ausführungsformen ist es auch möglich, dass die Messvorrichtung 10 und die Antriebsvorrichtung 50 durch eine Klickverbindung, eine Schnappverbindung oder durch eine Einrastverbindung lösbar miteinander verbunden werden können.

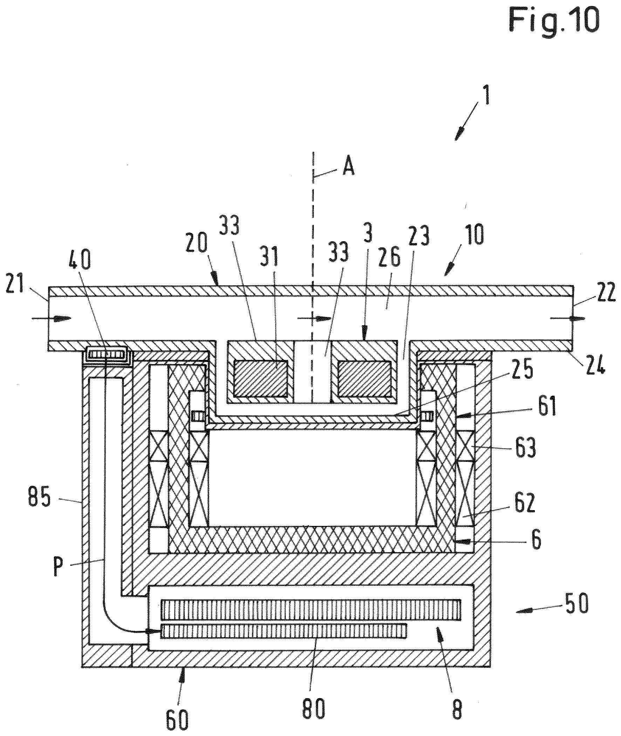

Fig. 10 zeigt in einer zu Fig.5 analogen Darstellung eine zweite Variante des ersten Ausführungsbeispiels. Im Folgenden wird nur auf die Unterschiede zu den bisher beschriebenen Ausführungsformen eingegangen. Es versteht sich, dass alle vorangehenden Erläuterungen in gleicher Weise oder in sinngemäss gleicher Weise auch für die zweite Variante gelten.

Bei der zweiten Variante umfasst die Messvorrichtung 10 eine Speichereinheit 40, in welcher Kalibrierungsdaten und optional andere Kennzeichnungsdaten gespeichert sind, die spezifisch für diese Messvorrichtung 10 sind. Die Antriebsvorrichtung 50 umfasst eine Schnittstelle 85, über welche Daten aus der Speichereinheit 40 an die Kontrolleinheit 8 übermittelbar sind. Insbesondere für Messungen, die eine sehr hohe Präzision verlangen, ist es oft notwendig, dass das Viskosimeter 1 kalibriert wird. Hierzu sollte dann üblicherweise die Messvorrichtung 10 gemeinsam mit der Antriebsvorrichtung 50 kalibriert werden. Wird nun die Messvorrichtung 10, die beispielsweise als Einmalvorrichtung ausgestaltet ist, durch eine neue Messvorrichtung 10 ersetzt, so könnte es notwendig sein, das Viskosimeter 1, das nun diese neue Messvorrichtung 10 -eingesetzt in die ursprüngliche Antriebsvorrichtung 50- umfasst, neu zu kalibrieren. Beispielsweise können geringfügige Variationen in den geometrischen Abmessungen von Komponenten der Messvorrichtung, z. B. geringfügige Variationen im Aussendurchmesser des Rotors 3 oder in seinen magnetischen Eigenschaften, dazu führen, dass nach dem Austausch der Messvorrichtung 10 eine neue Kalibrierung notwendig wird. Solche Variationen sind beispielsweise fertigungstechnisch bedingt und üblicherweise nicht vermeidbar, zumindest nicht mit einem vertretbaren Aufwand.

Um dieses Problem zu lösen, kann beispielsweise jede Messvorrichtung 10 in einem Referenzviskosimeter mit einer Referenz-Antriebsvorrichtung kalibriert werden. Die daraus resultierenden, für die jeweilige Messvorrichtung 10 spezifischen Kalibrierungsdaten werden dann in der Speichereinheit 40 dieser Messvorrichtung 10 gespeichert. Wird nun diese Messvorrichtung 10 in die Antriebsvorrichtung 50 eingesetzt, so werden die spezifischen Kalibrierungsdaten über die Schnittstelle 85 an die Kontrolleinheit 8 der Antriebsvorrichtung 50 übermittelt, sodass eine erneute Kalibrierung des Viskosimeters 1 nach dem Austausch der Messvorrichtung 10 nicht mehr notwendig ist.

Die Kontrolleinrichtung 8 umfasst eine Speicherleseeinheit 80, die ebenfalls im Antriebsgehäuse 60 angeordnet ist und die mit der Schnittstelle 85 signalverbunden ist. Somit können die in der Speichereinheit 40 gespeicherten Kalibrierungsdaten für die Messvorrichtung 10 über die Schnittstelle 85 ausgelesen und an die Speicherleseeinheit 80 der Kontrollvorrichtung 8 übertragen werden. Dieser Datenfluss ist in Fig. 10 durch den Pfeil mit dem Bezugszeichen P dargestellt.

Da die Messvorrichtung 10 vorzugsweise gamma-sterilisierbar ausgestaltet ist, wird auch die Speichereinheit 40 vorzugsweise gammastabil ausgestaltet, das heisst die Speichereinheit 40 ist so ausgestaltet, dass sie bei der Gamma-Sterilisierung keinen Schaden nimmt und insbesondere keine Kalibrierungsdaten verliert. Als gammastabile Speichereinheiten 40 sind beispielsweise geeignet: FRAM (Ferroelectric Random Access Memory), RFID Elemente (RFID: Radio Frequency Identification), oder optoelektronisch lesbare Elemente wie Barcodes oder zweidimensionale Codes, z.B. QR Codes (QR: Quick Response).

Fig. 11 zeigt in einer zu Fig. 6 analogen Darstellung ein zweites Ausführungsbeispiel eines erfindungsgemässen Viskosimeters 1, das sich von dem ersten Ausführungsbeispiel in der Ausgestaltung des elektromagnetischen Drehantriebs 90 unterscheidet. Im Folgenden wird nur auf die Unterschiede zu dem ersten Ausführungsbeispiel eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel bzw. seinen Varianten. Insbesondere haben die Bezugszeichen die gleiche Bedeutung wie sie bereits im Zusammenhang mit dem ersten Ausführungsbeispiel und seinen Varianten erläutert sind. Es versteht sich, dass alle vorangehenden Erläuterungen des ersten Ausführungsbeispiels und seiner Varianten in gleicher Weise oder in sinngemäss gleicher Weise auch für das zweite Ausführungsbeispiel gelten.

Bei dem zweiten Ausführungsbeispiel ist der elektromagnetische Drehantrieb 90 mit nur einem Wicklungssystem ausgestaltet, das sechs konzentrierte Wicklungen 65 umfasst. An dem Längsschenkel 612 jedes Spulenkerns 61 ist genau eine konzentrierte Wicklung 65 angeordnet. Bei dem zweiten Ausführungsbeispiel wird die Antriebs- und Lagerfunktion mit nur einem einzigen Wicklungssystem, nämlich mit den sechs konzentrierten Wicklungen 65 realisiert, sodass es also keine separaten Antriebs- und Steuerspulen wie bei dem ersten Ausführungsbeispiel gibt. Dabei kann die Antriebs- und Lagerfunktion so realisiert werden, dass die von der Kontrolleinrichtung 8 jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 65 eingeprägt wird.

Im Folgenden werden nun noch einige Ausführungsformen von Komponenten beschrieben, welche sowohl für das erste Ausführungsbeispiel als auch für das zweite Ausführungsbeispiel verwendet werden können.

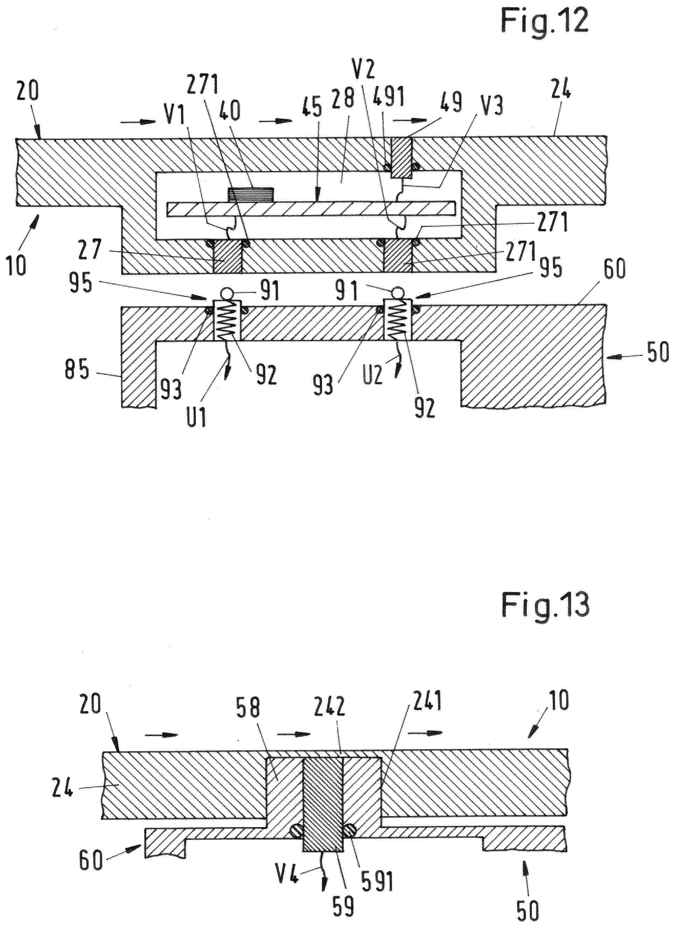

Fig. 12 zeigt eine mögliche Ausführungsform für eine Signalverbindung zwischen der Messvorrichtung 10 und der Antriebsvorrichtung 50, um beispielsweise Kalibrierungsdaten aus der Speichereinheit 40 der Messvorrichtung 10 in die Kontrolleinrichtung 8 der Antriebsvorrichtung 50 zu übertragen. Die in Fig. 12 dargestellte Signalverbindung ist als elektrische Signalverbindung ausgestaltet. Die Signalverbindung ist vorzugsweise so ausgestaltet, dass sie problemlos ein mehrfaches Austauschen der Messvorrichtung 10 übersteht.

In derjenigen Oberfläche des Antriebsgehäuses 60 der Antriebsvorrichtung 50, welche im zusammengesetzten Zustand, also wenn die Messvorrichtung 10 in die Antriebsvorrichtung 50 eingesetzt ist, der Messvorrichtung 10 zugewandt ist, sind einer oder mehrere Federkontakte 95 vorgesehen, welche jeweils einen aus der Oberfläche des Antriebsgehäuses 60 herausstehenden leitfähigen Kopf 91 umfassen, welcher durch ein Federelement 92 federbelastet ist. Bei der in Fig. 12 dargestellten Ausführungsform sind mit beispielhaftem Charakter zwei solche Federkontakte 95 dargestellt. Es versteht sich, dass in anderen Ausführungsformen auch nur ein Federkontakt 95 oder mehr als zwei Federkontakte 95 vorgesehen sein können. Jeder der Federkontakte 95 ist mittels einer elektrischen Signalverbindung U1 bzw. U2 mit der Kontrolleinrichtung 8, beispielsweise mit der Speicherleseeinheit 80 der Kontrolleinrichtung 8, signalverbunden. Der Kopf 91 der Federkontakte 95 ist vorzugsweise aus Gold gefertigt.

Im Boden 24 des Messgehäuses 20 der Messvorrichtung 10 sind elektrische Kontakte 27 vorgesehen, welche so angeordnet sind, dass sie im zusammengesetzten Zustand, also wenn die Messvorrichtung 10 in die Antriebsvorrichtung 50 eingesetzt ist, auf die Federkontakte 95 drücken und somit eine elektrische Verbindung zwischen der Messvorrichtung 10 und der Antriebsvorrichtung 50 bilden.

Die elektrischen Kontakte 27 sind jeweils durch ein Dichtungselement 271, beispielsweise einen O-Ring gegen das Austreten des Fluids aus der Messvorrichtung 10 geschützt. Es ist auch möglich, die elektrischen Kontakte 27 durch eine Klebeverbindung in dichtender Weise an dem Messgehäuse 20 zu befestigen, oder die elektrischen Kontakte 27 bei der Herstellung der Messvorrichtung 10, die vorzugsweise mit einem Spritzgussverfahren erfolgt, mit dem Kunststoff zu umspritzen.

Die Federkontakte 95 sind jeweils durch ein Dichtungselement 93, beispielsweise einen O-Ring, gegen das Eindringen des Fluids in die Antriebsvorrichtung 50 geschützt. Das Fluid ist auch in Fig. 12 durch die Pfeile ohne Bezugszeichen dargestellt

Im Boden 24 der Messvorrichtung 10 ist darstellungsgemäss oberhalb der elektrischen Kontakte 27 eine Kavität 28 vorgesehen, die als geschlossener Hohlraum ausgestaltet ist. In der Kavität 28 ist ein Speicher-PCB 45 vorgesehen auf welchem die Speichereinheit 40 angeordnet ist. Das Speicher-PCB 45 ist über elektrische Signalverbindungen V1, V2 mit den elektrischen Kontakten 27 im Boden 24 des Messgehäuses 20 verbunden. Die elektrischen Signalverbindungen V1 und V2 können auch als ein direkter körperlicher Kontakt zwischen dem Speicher-PCB 45 und den elektrischen Kontakten 27 ausgestaltet sein.

Die elektrischen Signalverbindungen U1, U2 in der Antriebsvorrichtung 50 sowie die elektrischen Signalverbindungen V1 und V2 können jeweils als Draht ausgestaltet sein.

Das Speicher-PCB 45 kann kommunikationsfähig und/oder zum Bereitstellen von elektrischer Energie ausgestaltet sein. Beispielsweise kann das Speicher-PCB 45 als Teil eines Bus-Systems ausgestaltet sein, z.B. für ein SPI (Serial Peripheral Interface) oder für einen I²C Bus (I2C: Inter-Integrated Circuit).

Optional, aber bevorzugt, umfasst die Messvorrichtung 10 einen Temperatursensor 49, mit welchem die Temperatur des Fluids in der Messvorrichtung 10 ermittelt werden kann. Da die Viskosität von sehr vielen Fluiden eine deutliche Temperaturabhängigkeit hat, ist es vorteilhaft, die Temperatur des Fluids möglichst nahe an dem Ort zu bestimmen, an dem auch die Viskosität des Fluids bestimmt wird.

Wie dies in Fig. 12 dargestellt ist, kann der Temperatursensor 49 in der Kavität 28 angeordnet werden, derart, dass er sich vollständig durch die Wandung hindurch erstreckt, welche die Kavität 28 begrenzt und welche im Betriebszustand von dem Fluid überströmt wird. Somit ist der Temperatursensor 49 im Betriebszustand im direkten körperlichen Kontakt mit dem Fluid, was eine besonders genaue Bestimmung der Temperatur des Fluid ermöglicht.

Da der Temperatursensor 49 andererseits in bzw. an der Kavität 28 angeordnet ist, kann er über eine elektrische Signalverbindung V3 mit dem Speicher-PCB 45 verbunden werden, sodass das Speicher PCB 45 den Temperatursensor 49 mit Energie versorgen und Messsignale von dem Temperatursensor 49 empfangen kann.

Der Temperatursensor 49 kann auf verschiedene Arten im Boden 24 der Messvorrichtung 10 fixiert werden. Beispielsweise kann er in einem dafür vorgesehen Loch durch Kleben oder durch einen Press-Fit fixiert werden. Auch ist es möglich, bei der Herstellung der Messvorrichtung 10, die vorzugsweise mit einem Spritzgussverfahren erfolgt, den Temperatursensor 49 mit dem Kunststoff zu umspritzen. Je nach Befestigung des Temperatursensors 49 im Boden 24 der Messvorrichtung 20 kann es vorteilhaft sein, an dem Temperatursensor 49 ein Dichtungselement 491, beispielsweise einen O-Ring, vorzusehen, um ein Eindringen des Fluids in die Kavität 28 zu verhindern.

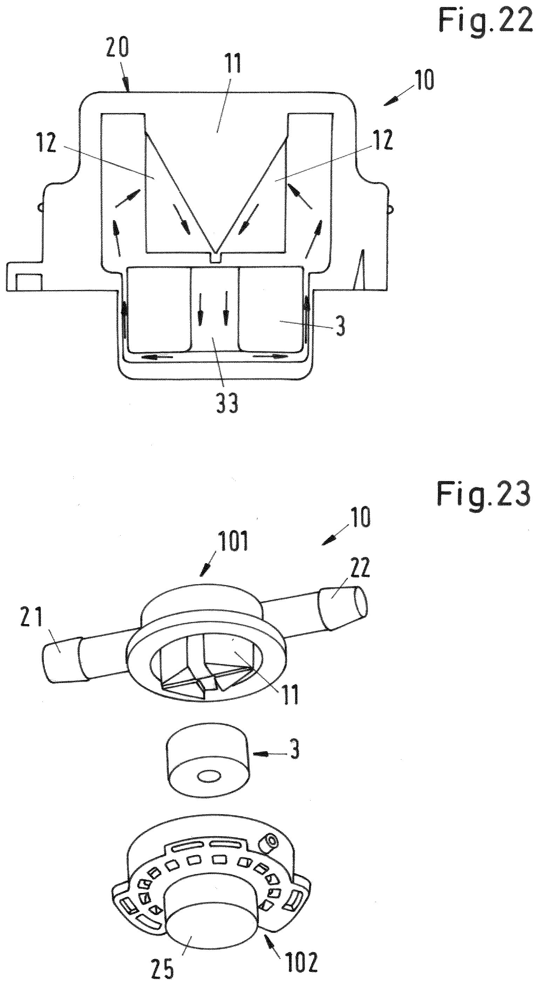

Natürlich ist es auch möglich, den Temperatursensor 49 an anderen Stellen der Messvorrichtung 10 bzw. des Messgehäuses 20 anzuordnen, beispielsweise an oder in einem Oberteil 101 (Fig. 23) oder am Einlass 21 oder am Auslass 22. Je nach Ausgestaltung kann es vorteilhaft sein, den Temperatursensor 49 über eine separate Signalleitung direkt mit der Kontrolleinrichtung 8 zu verbinden.

Eine weitere Möglichkeit besteht darin, die Messvorrichtung 10 so auszugestalten, dass sie zur Bestimmung der Temperatur des Fluids mit einem externen Temperatursensor in thermischen Kontakt gebracht werden kann, wobei der externe Temperatursensor nicht Bestandteil der Messvorrichtung 10 ist. Dies kann beispielsweise so realisiert werden, dass am Einlass 21 oder am Auslass 22 eine metallische Kontaktfläche, z. B. eine Metallhülse vorgesehen ist, welche einerseits in thermischen Kontakt mit dem die Messvorrichtung 10 durchströmenden Fluid ist, und welche andererseits mit einem externen Temperatursensor in thermischen Kontakt gebracht werden kann. Der externe Temperatursensor ist mit der Kontrolleinrichtung 8 signalverbunden. Die metallische Kontaktfläche bzw. die Metallhülse kann beispielsweise in einer Ausnehmung am Einlass 21 oder bevorzugt am Auslass 22 angeordnet sein, in welche der externe Temperatursensor eingeschoben werden kann, oder in welcher der externe Temperatursensor in anderer Weise fixierbar ist.

Prinzipiell sind alle an sich bekannten Temperatursensoren 49 für das Viskosimeter 1 geeignet. Im Hinblick auf eine möglichst hohe Flexibilität bezüglich der Fluid ist der Temperatursensor 49 vorzugsweise korrosionsbeständig ausgestaltet. Ferner ist ein metallischer Temperatursensor 49 bevorzugt.

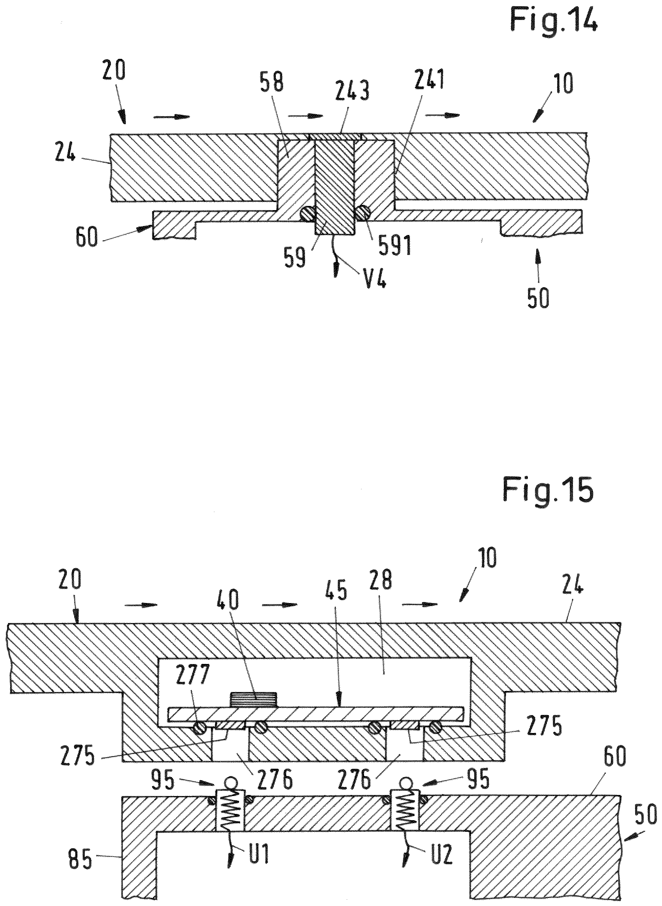

Alternativ oder zusätzlich kann auch in der Antriebsvorrichtung 50 ein Temperatursensor 59 zur Erfassung der Temperatur des Fluids in der Messvorrichtung 10 vorgesehen sein. Fig. 13 und Fig. 14 zeigen jeweils in einer Detaildarstellung eine solche Anordnung des Temperatursensors 59 in der Antriebsvorrichtung 50. Bezüglich Auswahl und Befestigung des Temperatursensors 59 gilt sinngemäss Gleiches wie es vorangehend für den Temperatursensor 49 in der Messvorrichtung 10 erläutert wurde.

Auch der in der Antriebsvorrichtung 50 vorgesehene Temperatursensor 59 ist so angeordnet, dass er im Betriebszustand möglichst nahe an dem die Messvorrichtung 10 durchströmenden Fluid ist und in Wärmekontakt mit dem Fluid treten kann.

Bei den in Fig. 13 und Fig. 14 dargestellten Anordnungen ist der Temperatursensor 59 jeweils in derjenigen Wandung des Antriebsgehäuses 60 platziert, welche im zusammengesetzten Zustand, das heisst, wenn die Messvorrichtung 10 in die Antriebsvorrichtung 50 eingesetzt ist, dem Messgehäuse 10 zugewandt ist bzw. an das Messgehäuse 10 angrenzt oder anstösst. In dieser Wandung des Antriebsgehäuses 60 ist eine Aufnahme 58 für den Temperatursensor 59 vorgesehen. Die Aufnahme 58 ist derart ausgestaltet, dass sie aus der Wandung hervorsteht. Der Temperatursensor 59 ist über eine elektrische Signalverbindung V4 mit der Kontrolleinrichtung 8 verbunden.

Je nach Befestigung des Temperatursensors 59 in der Aufnahme 58 kann es vorteilhaft sein, an dem Temperatursensor 59 ein Dichtungselement 591, beispielsweise einen O-Ring, vorzusehen, um ein Eindringen des Fluids in die Antriebsvorrichtung 50 zu verhindern. Es ist auch möglich, den Temperatursensor 59 durch einen Press-Fit oder eine Klebeverbindung in dichtender Weise an dem Antriebsgehäuse 60 zu befestigen, oder den Temperatursensor 59 bei der Herstellung des Antriebsgehäuses 60, die vorzugsweise mit einem Spritzgussverfahren erfolgt, mit dem Kunststoff zu umspritzen.

Im Boden 24 der Messvorrichtung 10 ist ein Loch 241 vorgesehen, dass derart ausgestaltet und angeordnet ist, dass die Aufnahme 58 passgenau in das Loch 241 eingreift, wenn die Messvorrichtung 10 in die Antriebsvorrichtung 50 eingesetzt ist.

Bei der in Fig. 13 dargestellten Ausführungsform ist das Loch 241 so ausgestaltet, dass es den Boden 24 des Messgehäuses 20 nicht vollständig durchdringt, sondern dass ein dünner Bereich des Bodens 24 als Trennwand 242 vorhanden ist, die im Betriebszustand das strömende Fluid, welches auch in den Fig. 13 und Fig. 14 jeweils durch die Pfeile ohne Bezugszeichen dargestellt ist, von der Aufnahme 58 trennt. Die Dicke der Trennwand 242 ist dabei so bemessen, dass die Trennwand 242 einerseits noch eine ausreichende Stabilität aufweist und andererseits einen guten thermischen Kontakt zwischen dem Temperatursensor 59 und dem Fluid ermöglicht. Optional ist es möglich, den Temperaturabfall über die Trennwand 242 (die vorzugsweise als Teil des Antriebsgehäuses 60 auch aus einem Kunststoff besteht) durch einen Korrekturalgorithmus zu berücksichtigen bzw. zu kompensieren.

Bei der in Fig. 14 dargestellten Ausführungsform ist das Loch 241 so ausgestaltet, dass es den Boden 24 des Messgehäuses 60 vollständig durchdringt. An dem Ende des Lochs 241, das im Betriebszustand von dem Fluid überströmt wird, ist ein thermisches Kopplungselement 243 angeordnet, welches das Loch 241 verschliesst und vorzugsweise dichtend verschliesst. Im Betriebszustand ist das thermische Kopplungselement 243 auf der einen Seite in direkten körperlichen Kontakt mit dem Fluid und auf der anderen Seite in direkten körperlichen Kontakt mit dem Temperatursensor 59.

Das thermische Kopplungselement 243 besteht vorzugsweise aus einem korrosionsbeständigen Metall, das eine gute Wärmeleitfähigkeit aufweist oder aus einem anderen korrosionsbeständigen Material, das eine gute Wärmeleitung aufweist. Durch das thermische Kopplungselement 243 lässt sich der Wärmekontakt zwischen dem Fluid und dem Temperatursensor 59 verbessern.

Das thermische Kopplungselement 243 kann auf verschiedene Arten im Boden 24 der Messvorrichtung 10 fixiert werden. Beispielsweise kann es in dem Loch 241 durch Kleben oder durch einen Press-Fit fixiert werden. Auch ist es möglich, bei der Herstellung der Messvorrichtung 10, die vorzugsweise mit einem Spritzgussverfahren erfolgt, das thermische Kopplungselement 243 mit dem Kunststoff zu umspritzen. Optional kann auch an dem thermischen Kopplungselement 243 ein Dichtungselement (nicht dargestellt), beispielsweise ein O-Ring vorgesehen sein.

Fig. 15 zeigt in einer zu Fig. 12 analogen Darstellung eine weitere Ausführungsform für eine Signalverbindung, welche eine elektrische Verbindung zwischen der Messvorrichtung 10 und der Antriebsvorrichtung 50 ermöglicht. Allerdings ist in Fig. 15 der Temperatursensor 49, der natürlich auch bei der in Fig. 15 dargestellten Ausführungsform vorgesehen sein kann, nicht dargestellt.

Bei der in Fig. 15 dargestellten Ausführungsform ist anstelle der elektrischen Kontakte 27 in der Messvorrichtung 10 (Fig. 12) das Speicher-PCB 45 mit elektrischen Kontaktflächen 275 versehen, die jeweils über einer Öffnung 276 angeordnet ist, sodass jeder Federkontakt 95 eine der elektrischen Kontaktflächen 275 kontaktieren kann. Das Speicher-PCB 45 kann auch als flexibles PCB ausgestaltet sein. Das Speicher-PCB 45 kann beispielsweise mittels Verklebung oder mittels Press-Fit Technologie in der Kavität 28 wasserdicht befestigt werden, sodass das Fluid nicht durch die Kavität 28 aus der Messvorrichtung 10 herausgelangen kann. Ferner können um die Öffnungen 276 herum Dichtungselemente 277, beispielsweise jeweils ein O-Ring, vorgesehen sein.

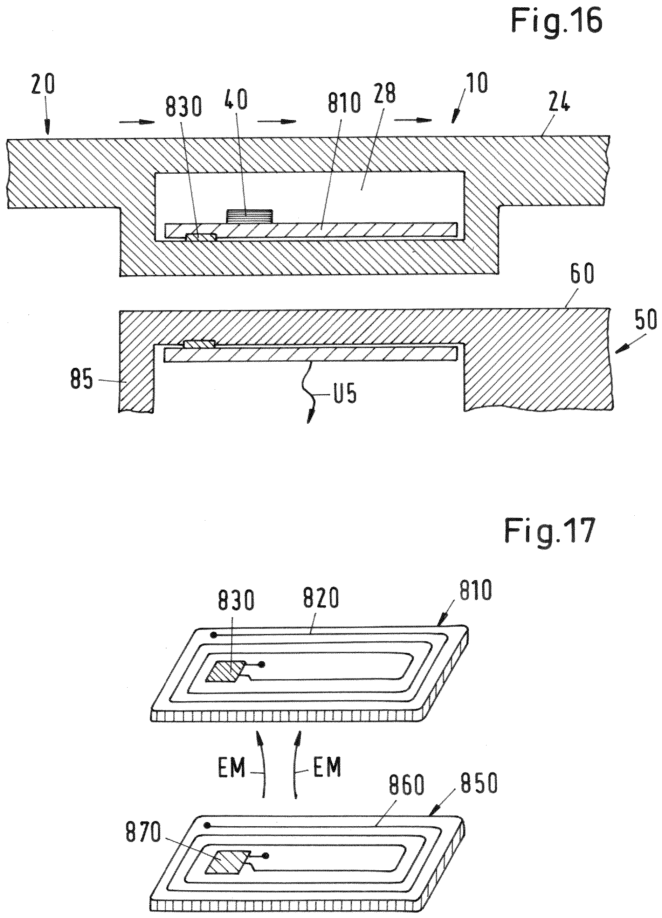

Die Kommunikation zwischen der Speichereinheit 40 der Messvorrichtung 10 und der Kontrolleinrichtung 8 der Antriebsvorrichtung 50 kann auch mittels RFID Technologie erfolgen (RFID: Radio Frequency Identification). Eine solche Ausgestaltung ist in Fig. 16 dargestellt. Zum besseren Verständnis zeigt Fig. 17 noch die beiden Komponenten für die Radiofrequenz Identifikation.

In der Messvorrichtung 10 ist ein erster Antennenträger 810 vorgesehen, der eine erste Antenne 820 sowie die Speichereinheit 40 (in Fig. 17 nicht dargestellt) trägt, wobei die Antenne 820 mit einem passiven Transponder 830 verbunden ist, der auf dem ersten Antennenträger 810 angeordnet ist.

In der Antriebsvorrichtung 50 ist ein zweiter Antennenträger 850 vorgesehen, der eine zweite Antenne 860 trägt, welche mit einem aktiven Transceiver 870 verbunden ist, der auf dem zweiten Antennenträger 850 angeordnet ist. Der aktive Transceiver 870 ist über eine Signalverbindung U5 mit der Kontrolleinrichtung signalverbunden.

Der passive Transponder 830 und der aktive Transceiver 870 kommunizieren in an sich bekannter Weise über elektromagnetische Felder EM miteinander.

Der passive Transponder 830 und der aktive Transponder 870 sind jeweils so angeordnet, dass sie im zusammengesetzten Zustand der Messvorrichtung 10 und der Antriebsvorrichtung 50 möglichst dicht aneinander sind. Durch eine geeignete EM Feldstärke und Form der Antennen 820, 860 kann erreicht werden, dass nur der direkt benachbarte passive Transponder 830 ausgelesen wird und keine weiteren in der nahen Umgebung.

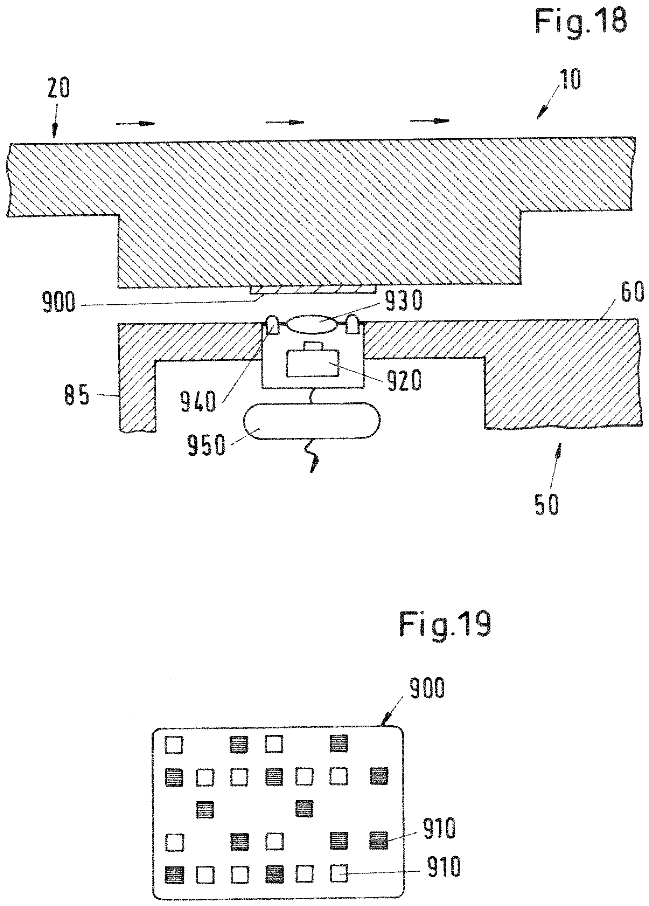

Eine weitere Möglichkeit, Kalibrierungsdaten oder Konfigurationsparameter einer spezifischen Messvorrichtung 10 an die Speicherleseeinheit 80 der Kontrolleinrichtung 8 der Antriebsvorrichtung 50 zu übertragen, ist in Fig. 18 und in Fig. 19 dargestellt. Bei dieser Ausführungsform ist die Messvorrichtung 10 mit einer Kennzeichnung (tag) 900 versehen, welche die spezifischen Informationen 910, also beispielsweise die Kalibrierungsdaten, für diese Messvorrichtung 10 enthält. Die Kennzeichnung 900 ist beispielsweise als zweidimensionaler "Bar Code" ausgestaltet, z.B. als ein QR Code. Zum besseren Verständnis zeigt Fig. 19 ein Beispiel für eine solche Kennzeichnung 900.

Die Kennzeichnung 900 ist von aussen sichtbar bzw. von aussen optisch zugänglich auf der Messvorrichtung angebracht, und zwar auf derjenigen Seite der Messvorrichtung, welche im zusammengesetzten Zustand der Antriebsvorrichtung 60 zugewandt ist.

In der Antriebsvorrichtung 50 ist eine Kamera 920 vorgesehen, welche die Kennzeichnung 900 erfassen kann. Das heisst, die Kennzeichnung 900 ist derart auf der Messvorrichtung 10 angeordnet, dass sie sich im zusammengesetzten Zustand, also wenn die Messvorrichtung 10 in die Antriebsvorrichtung 50 eingesetzt ist, in der Sichtlinie der Kamera 920 befindet.

Die Kamera 920 ist mit einer Verarbeitungseinheit 950 signalverbunden, die als separate Einheit ausgestaltet sein kann, oder die in die Speicherleseeinheit 80 der Kontrolleinrichtung 8 integriert sein kann. Optional können in der Antriebsvorrichtung 50 eine Linse 930 und/oder mindestens eine Beleuchtungsquelle 940 vorgesehen sein, um eine zuverlässige optische Erfassung der Kennzeichnung 900 durch die Kamera 920 unter allen Bedingungen zu gewährleisten. Die Beleuchtungsquelle 940 kann insbesondere als LED ausgestaltet sein.

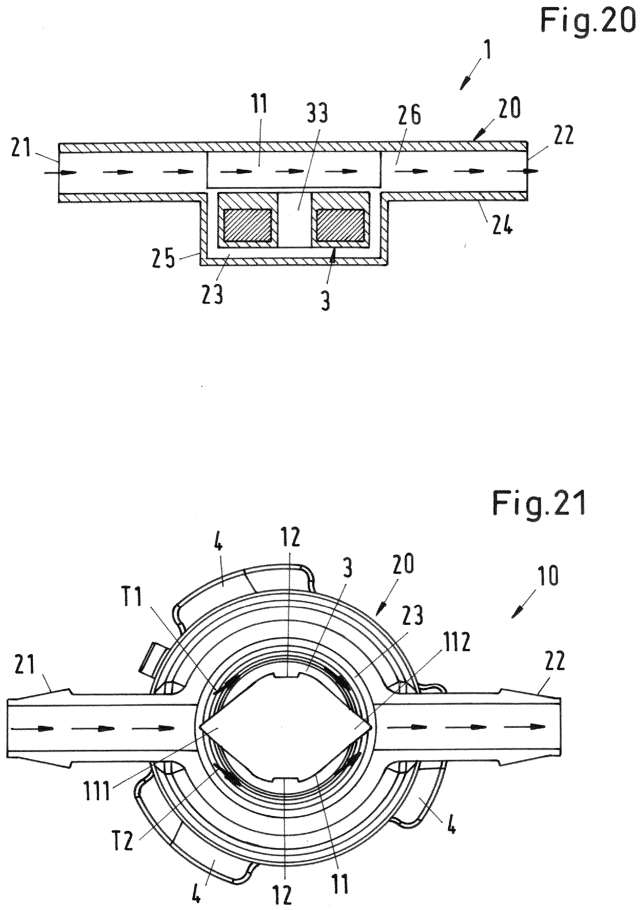

Fig. 20 zeigt in einer schematischen Schnittdarstellung eine Variante für die Messvorrichtung 10. Diese Variante eignet sich für alle bisher beschriebenen Ausführungsbeispiele bzw. kann mit allen Ausführungsbeispielen und Ausführungsformen kombiniert werden.

Zum besseren Verständnis zeigt Fig. 21 eine Aufsicht auf diese Variante, wobei die Variante oben geöffnet dargestellt ist, sodass die Messkammer 23 sichtbar ist. Fig. 22 zeigt eine Schnittdarstellung der Variante in einem Schnitt entlang der axialen Richtung A, wobei die Blickrichtung vom Einlass 21 in die Hauptströmungsverbindung 26 gerichtet ist. Fig. 23 zeigt eine perspektivische Explosionsdarstellung der Variante

Bei der Variante für die Messvorrichtung 10 ist in der Hauptströmungsverbindung 26 ein Flussleitelement 11 vorgesehen ist, welches zentral über dem Rotor 3 angeordnet ist, und welches zum Aufteilen des Fluids in einen ersten Teilfluss T1 und in einen zweiten Teilfluss T2 ausgestaltet ist, derart dass das Flussleitelement 11 zwischen dem Einlass 21 und dem Auslass 22 auf einer Seite von dem ersten Teilfluss T1 umströmt wird, und auf der anderen Seite von dem zweiten Teilfluss T2 umströmt wird. Dabei ist das Flussleitelement 11 vorzugsweise so ausgestaltet, dass es das Fluid möglichst symmetrisch in die beiden Teilflüsse T1 und T2 aufteilt.

Wie dies insbesondere die Aufsicht in Fig. 21 zeigt, hat das Flussleitelement 11 zwei Endbereiche 111, 112, nämlich einen ersten Endbereich 111, welcher dem Einlass 21 zugewandt ist, und welcher sich bis kurz vor den Einlass 21 erstreckt, sowie einen zweiten Endbereich 112, welcher dem Auslasszugewandt ist, und welcher sich bis kurz vor den Auslass 22 erstreckt. Der erste Endbereich 111 ist sich in radialer Richtung erweiternd ausgestaltet, derart das der erste Endbereich 111 in radialer Richtung gesehen breiter wird, wenn man sich vom Einlass 21 in Richtung Auslass 22 bewegt. Der zweite Endbereich 112 ist sich in radialer Richtung verjüngend ausgestaltet, derart das der zweite Endbereich 112 in radialer Richtung gesehen schmäler wird, wenn man sich vom Einlass 21 auf den Auslass 22 zu bewegt. Durch diese Ausgestaltung wird das Fluid in Strömungsrichtung gesehen unmittelbar hinter dem Einlass 21 in die beiden Teilflüsse T1, T2 aufgeteilt, und unmittelbar vor dem Auslass 22 werden dies beiden Teilflüsse T1, T2 wieder zusammengeführt.

Durch das Flussleitelement 11, welches den zentralen Bereich des Rotors 3 überdeckt, wird das Fluid also im Betriebszustand in die beiden Teilflüsse T1 und T2 aufgeteilt, welche im Wesentlichen nur noch über die Peripherie der Oberfläche des Rotors 3 strömen. Somit werden mittels des Flussleitelements 11 Rotationsströmungen in der Hauptströmungsverbindung 26 verhindert oder zumindest drastisch reduziert. Solche Rotationsströmungen können sich negativ auswirken, weil sie an der Oberfläche des Rotors 3 auf der einen Seite der Hauptströmung entgegen fliessen und auf der anderen Seite in der gleichen Richtung strömen wie die Hauptströmung. Hierdurch können asymmetrische Reibungseffekte entstehen, welche sich negativ auf die Messung der Viskosität auswirken können.

Ein weiterer Vorteil der Ausgestaltung mit dem Flussleitelement 11 besteht darin, dass dieses einen signifikanten Raum in der Hauptströmungsverbindung 26 in Anspruch nimmt. Dies hat den Vorteil, dass das nasse Volumen, womit der Bereich der Messvorrichtung 10 gemeint ist, welcher mit dem Fluid gefüllt ist, bzw. von dem Fluid durchströmt wird, reduziert wird. Dies ist insbesondere dann ein wichtiger Aspekt, wenn das Fluid ein sehr wertvolles oder teures Fluid ist.

Eine weitere bevorzugte Massnahme besteht darin, dass das Flussleitelement 11 eine Mehrzahl von Seitenkanälen 12 aufweist, welche einen Teil des Fluids aus der Hauptströmungsverbindung 26 in die axiale Richtung A auf den Rotor 3 hin umlenken. Durch diese Massnahme lässt sich der Sekundärstrom verstärken, welches der Strom des Fluids ist, der den Rotor 3 umströmt und somit einen wesentlichen Faktor für die Bestimmung der Viskosität darstellt.

Der Sekundärstrom ist in Fig. 22 durch die Pfeile ohne Bezugszeichen dargestellt. In Fig. 22 ist zudem auch die Ausgestaltung der Seitenkanäle 12 gut zu erkennen. Fig. 22 zeigt einen Schnitt in axialer Richtung A durch die Messvorrichtung 10, wobei der Schnitt in der Mitte zwischen dem Einlass 21 und dem Auslass 22 erfolgt. Der Blick ist vom Einlass 21 auf die Messkammer 23 gerichtet. In dieser Darstellung der Fig. 22 hat jeder Seitenkanal 12 ein dreieckiges Profil, wobei die in radialer Richtung gemessene Tiefe jedes Seitenkanals 12 zunimmt, wenn man sich in axialer Richtung auf den Rotor 3 zubewegt.

Durch die Seitenkanäle 12 wird ein Teil des Fluids als Sekundärstrom aus der radialen Richtung in die axiale Richtung umgelenkt und strömt durch das dreieckige Profil der Seitenkanäle 12 bedingt primär durch die zentrale Öffnung 33 des Rotors 3. Nach Durchströmen der zentralen Öffnung 33 fliesst der Sekundärstrom entlang der Unterseite des Rotors 3 und wird dann zwischen der die Ausstülpung 25 begrenzenden Wandung und der Mantelfläche des Rotors 3 in axialer Richtung A zurück in die Hauptströmungsverbindung 26 geführt.

Durch das Flussleitelement 11 mit den Seitenkanälen 12 wird der Sekundärstrom des Fluids verstärkt wodurch sich der Fluidaustauch am Rotor 3 verbessert.

Vorzugsweise wird die Messvorrichtung 10 aus drei Hauptkomponenten zusammengesetzt (siehe Fig. 23), nämlich dem Rotor 3, welcher den magnetisch wirksamen Kern 31 sowie die Ummantelung 32 umfasst, einem Oberteil 101, welches den oberen Teil des Messgehäuses 20, den Einlass 21, den Auslass 22 und den Strömungsleitkörper 11 umfasst, sowie einem Unterteil 102, welches den Boden 24 mit der Ausstülpung 25 und der Messkammer 23 umfasst.

Insbesondere das Oberteil 101 und das Unterteil 102 werden vorzugsweise in einem Spritzgussverfahren hergestellt und anschliessend mit an sich bekannten Methoden zusammengefügt, nachdem der Rotor 3 in der Messkammer 23 platziert wurde. Als Verfahren zum Zusammenfügen von Oberteil 101 und Unterteil 102 eignen sich insbesondere Schweissverfahren wie beispielsweise Laserschweissen oder Infrarotschweissen oder auch Klebeverfahren.

Da das erfindungsgemässe Viskosimeter 1 eine sehr einfache, präzise und leicht zu handhabende Bestimmung der Viskosität eines Fluids ermöglicht, ist es insbesondere auch dazu geeignet, charakteristische Grössen, Eigenschaften oder Zustände eines Fluids zu bestimmen, die sich mit Hilfe der Viskosität des Fluids bestimmen lassen. Eine wichtige dieser Grössen ist die Konzentration einer Komponente in einem Fluid. Daher wird durch die Erfindung ferner ein Verfahren zur Bestimmung einer Konzentration einer Komponente in einem Fluid vorgeschlagen, das durch die folgenden Schritte gekennzeichnet ist:
- Bereitstellen eines Viskosimeters 1, welches gemäss der Erfindung ausgestaltet ist,
- Bereitstellen eines Zusammenhangs zwischen der Konzentration der Komponente in dem Fluid und der Viskosität des Fluids,
- Bestimmen der Viskosität des Fluids mittels des Viskosimeters 1,
- Bestimmen der Konzentration aus dem Zusammenhang zwischen der Konzentration und der Viskosität.

Solche Konzentrationsbestimmungen spielen insbesondere auch in der Biotechnologie und in der pharmazeutischen Industrie eine wichtige Rolle. Als Beispiele seien hier die Konzentrationen von Proteinen beispielsweise in einem Bioreaktor oder in eine Zellbrühe genannt.

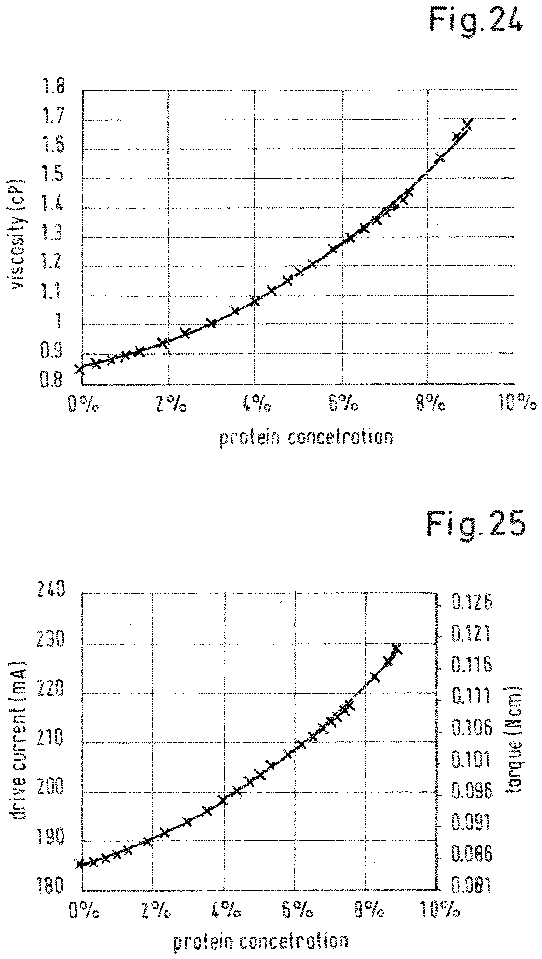

Mit beispielhaftem Character zeigt Fig. 24 den Zusammenhang zwischen der auf der horizontalen Achse aufgetragenen Konzentration eines Proteins in einem Fluid und der auf der vertikalen Achse aufgetragenen Viskosität des Fluids. Es ist sehr deutlich zu erkennen, dass aufgrund dieses Zusammenhangs zwischen der Konzentration und der Viskosität eine Viskositätsmessung sehr gut geeignet ist um die Proteinkonzentration in einem Fluid zu bestimmen.

Da die Viskosität in dem erfindungsgemässen Viskosimeter vorzugsweise mittels des Antriebsstroms bestimmt wird, welcher zum Antreiben der Rotation des Rotors 3 benötigt wird, ist in Fig. 25 noch der Zusammenhang gezeigt zwischen der Konzentration des Proteins (horizontale Achse) und dem Antriebsstrom (linke vertikale Ache) bzw. dem Drehmoment (rechte vertikale Achse), welches für das Antreiben der Rotation des Rotors 3 benötigt wird. Auch hier ist zu erkennen, dass dieser Zusammenhang eine sehr gute Bestimmung der Konzentration eines Proteins in einem Fluid mit Hilfe des erfindungsgemässen Viskosimeters 1 ermöglicht.

Eine wichtige Anwendung ist beispielsweise die Bestimmung der Immunoglobulin (Antikörper) Konzentration in einem Fluid. Speziell die Bestimmung der Konzentration der Immunoglobuline spielt in der modernen Diagnostik, in der Biotechnologie und bei der Entwicklung von Impfstoffen und Medikamenten eine bedeutende Rolle. Für solche Anwendungen ist das erfindungsgemässe Viskosimeter 1 seht gut geeignet.

Da jedes Immunoglobulin eine charakteristische, Konzentrationsabhängigkeit der Viskosität zeigt, lässt sich bei bekannter Klasse des Immunoglobulins (IgG, IgM, IgA, IgD, IgE) und Unterklasse (z.B. igG1, igG2, igG3, igG4) der Immunoglobuline, die Immunoglobulinkonzentration aus der Viskosität bestimmen oder umgekehrt, bei Kenntnis der Konzentration, auf die Art der Immunoglobuline schliessen. Bei Mischungen von zwei Proteinen (z.B. igG1 und igG4) und Kenntnis der Gesamtproteinkonzentration aufgrund einer anderen physikalischen Grösse wie beispielsweise der Dichte der Flüssigkeit, der Ausbreitungsgeschwindigkeit von Ultraschall in der Flüssigkeit, der Absorption von Licht bei einer oder mehreren Wellenlängen oder des Refraktionsindexes von Licht bei einer oder mehreren Wellenlängen etc. lässt sich über die Viskosität das Mischverhältnis beziehungsweise der relative Anteil der beiden Proteine bestimmen.

Es versteht sich von selbst, dass sich mit derselben Methode auch die Mischverhältnisse anderer Flüssigkeiten verschiedener Viskosität bestimmen lassen.

Da der Zusammenhang zwischen der Viskosität und der Proteinkonzentration in einem Fluid im Allgemeinen von der Temperatur abhängig ist, wird vorzugsweise der Zusammenhang zwischen der Konzentration des Proteins in dem Fluid und der Viskosität des Fluids für mehrere Temperaturen ermittelt und dann beispielsweise als ein dreidimensionales Kennlinienfeld mit den Dimensionen Konzentration, Viskosität, Temperatur in der Kontrolleinrichtung 8 hinterlegt, z. B in Form einer Lookup-Tabelle. Natürlich ist es auch möglich, die Bestimmung der Konzentration auf einer externen Datenverarbeitungsanlage vorzunehmen, in welche die Messergebnisse oder Messwerte des Viskosimeters 1 eingespeist werden.

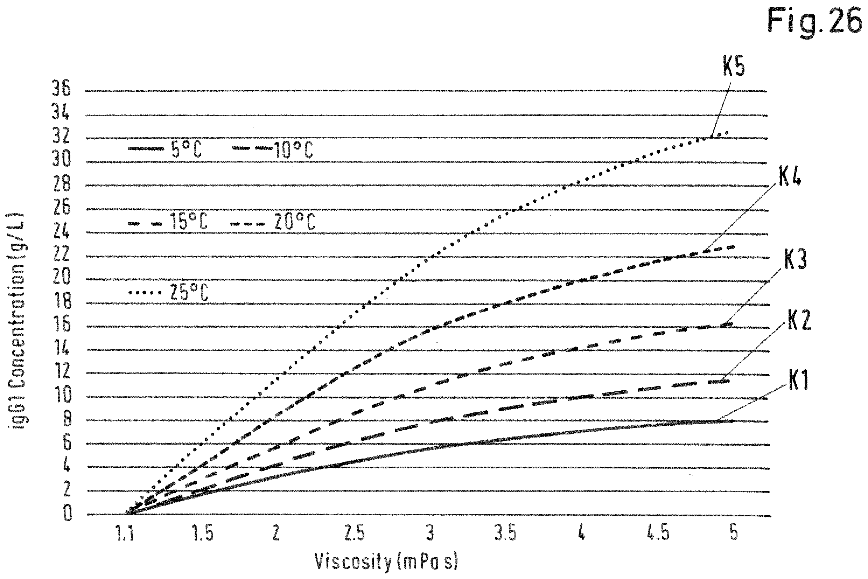

Als Beispiel für die Temperaturabhängigkeit des Zusammenhangs zwischen Konzentration und Viskosität ist in Fig. 26 der Zusammenhang zwischen der Konzentration des Immunoglobulins G1 (igG1) (vertikale Achse) in einem Fluid und der Viskosität des Fluids (horizontale Achse) für fünf verschiedene Temperaturen dargestellt. Die Kurve K1 zeigt den Zusammenhang für eine Temperatur von 5°C. Die Kurve K2 zeigt den Zusammenhang für eine Temperatur von 10°C. Die Kurve K3 zeigt den Zusammenhang für eine Temperatur von 15°C. Die Kurve K4 zeigt den Zusammenhang für eine Temperatur von 20°C. Die Kurve K5 zeigt den Zusammenhang für eine Temperatur von 25°C.

Ein solcher Zusammenhang wie er in Fig. 26 dargestellt ist, kann gemäss dem erfindungsgemässen Verfahren beispielsweise zur on-line Überwachung der Proteinkonzentration in verschiedenen Bioprozessverfahren, insbesondere bei Downstream-Bioprozessverfahren dienen. Beispiele für Downstream-Bioprozessverfahren, bei welchen die Überwachung der Proteinkonzentration oder auch direkt der Viskosität von Nutzen sein kann, sind beispielsweise Querstrom- oder Crossflow-Filtrationsverfahren, insbesondere Ultrafiltrations- und Diafiltrationsverfahren, welche zur Reinigung und/oder Aufkonzentration der biotechnologisch hergestellten Wirkstoffe dienen. Bei solchen Querstromfiltrationsverfahren kann die laufende Überwachung der Viskosität dazu dienen, andere Prozessparameter wie beispielsweise den optimalen Transmembrandruck und den Tangentialfluss zu ermitteln und an den Prozessfortschritt anzupassen. Die indirekte Bestimmung der Proteinkonzentration ist ein wichtiger Indikator für den Prozessfortschritt und für den Prozess-Yield (Prozessausbeute).

Bei Zentrifugationstrennverfahren, insbesondere bei kontinuierlichen Zentrifugationsverfahren, liefert die Viskosität ebenfalls entscheidende Hinweise über den Prozessfortschritt beziehungsweise die Prozesskonstanz.

Bei Chromatographieverfahren, insbesondere bei kontinuierlichen Chromatographieverfahren kann die Viskosität, und die daraus gewonnene Proteinkonzentration Aufschlüsse über den Prozess-Yield und den Zustand der Chromatographie Kolonen beziehungsweise der Chromatographie Membranen geben.

Da in Bioprozessanlagen eine grosse Anzahl von verschiedenen Proteinlösungen und andere Flüssigkeiten verarbeitet werden, kann das erfindungsgemässe Viskosimeter 1 ferner einen zusätzlichen Speicher umfassen oder für die Kommunikation mit einem externen Speicher ausgestaltet sein. In diesem Speicher können viele verschiedene Datensätze gespeichert sein, welche analog zu dem in Figur 26 dargestellten Zusammenhang zwischen Viskosität, Temperatur und Konzentration des Immunoglobulins G1 (igG1), den Zusammenhang zwischen Viskosität, Temperatur und Konzentration verschiedener Flüssigkeiten, insbesondere von Proteinlösungen, beschreiben. Die Datensätze können beispielsweise als Stützwerte in Lookup-Tabellen abgelegt werden, wobei die Temperatur und die Viskosität zusammen die unteren und die oberen "Bits" einer Speicheradresse ausmachen können, und wobei in der entsprechenden Speicherzelle der Konzentrationswert als Stützwert gespeichert wird.

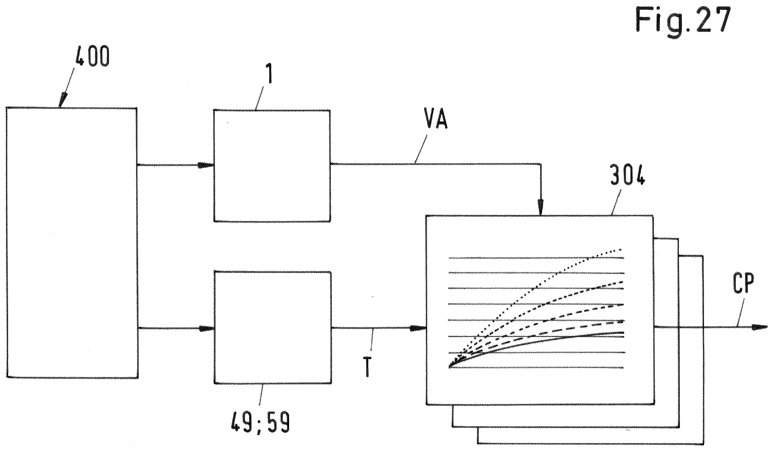

Diese Datensätze können entweder direkt in einem Datenspeicher des erfindungsgemässen Viskosimeters 1, der beispielsweise in der Kontrolleinrichtung 8 vorgesehen ist, oder in einer mit dem Viskosimeter verbundenen, separaten Auswerteeinheit 304 (z. B. Fig. 27) abgelegt werden.

Selbstverständlich ist es auch möglich, solche Datensätze auf einem Cloud-Speicher abzulegen und die Datensätze über eine Internet-Verbindung einer grossen Anzahl von erfindungsgemässen Viskosimetern 1 oder Auswerteeinheiten 304 zugänglich zu machen. Dadurch können die Datensätze laufend ergänzt und verbessert werden.

Ferner ist es möglich, dass die Datensätze auf Chip-Karten, auswechselbaren Flash-Memoryspeichern oder andern internen oder externen Speichermedien abgelegt werden. Unter anderem ist es auch möglich, die Daten in der Speichereinheit 40 der Messvorrichtung 10 zusammen mit den Kalibrierungsdaten der Messvorrichtung 10 abzuspeichern.

In diesem Fall wird die Messvorrichtung 10 vorzugsweise für die Konzentrationsmessung von einer Flüssigkeit oder von mehreren Flüssigkeiten konfiguriert. Insbesondere werden alle benötigten Kalibrierungsdaten in der Speichereinheit 40 der Messvorrichtung hinterlegt.

Zusammen mit mindestens einem der erwähnten Datenspeicher und den darin abgelegten Datensätzen, kann das erfindungsgemässe Viskosimeter 1 insbesondere auch als Konzentrationsmessgerät für verschiedene Flüssigkeiten, insbesondere für Proteinlösungen verwendet bzw. eingesetzt werden

Fig. 27 zeigt eine schematische Darstellung eines ersten Ausführungsbeispiels eines erfindungsgemässen Verfahrens, bei welchem eine Proteinkonzentration bestimmt wird. Bei diesem Ausführungsbeispiels des erfindungsgemässen Verfahrens wird also das Viskosimeter 1 als ein Konzentrationsmessgerät verwendet.

Bei diesem ersten Ausführungsbeispiel wird zumindest eine Proteinkonzentration in einer Flüssigkeit ermittelt. Das Viskosimeter 1 ist üblicherweise in ein Fluidsystem 400 integriert, welches Pumpen, Zuführungen, sowie Bioprozessgeräte, wie beispielsweise Filtervorrichtungen, Zentrifugen, Chromatographieanlagen, Bioreaktoren und/oder Mischanlagen umfassen kann, was in Fig. 27 nicht im Detail, sondern lediglich als das Fluidsystem 400 dargestellt ist.

Mittels des Viskosimeters 1 wird kontinuierlich oder in vorgebbaren zeitlichen Abständen eine aktuelle Viskosität VA der Flüssigkeit bestimmt, welche einer Auswerteeinheit 304 übermittelt wird. Mittels des Temperatursensors 49 und/oder 59 wird die Temperatur T der Flüssigkeit ermittelt. Auch wenn die Temperatur T vorzugsweise mit einem in dem Viskosimeter 1 angeordneten Temperatursensor bestimmt wird, also beispielsweise mit dem Temperatursensor 49 in der Messvorrichtung 10 oder mit dem Temperatursensor 59 in der Antriebsvorrichtung 50, so ist es auch möglich, die Temperatur T mit einer externen Temperaturmessvorrichtung zu bestimmen, die nicht in das Viskosimeter 1 integriert ist. In der Auswerteeinheit 304 sind, wie oben beschrieben, die Zusammenhänge zwischen der Temperatur T, der Viskosität und der Konzentration für mindestens eine oder für mehrere Flüssigkeiten hinterlegt, wie sie beispielhaft in Fig. 26 für das Immunoglobulin igG1 gezeigt sind. Die Auswerteeinheit 304 bestimmt dann aus der Viskosität und der Temperatur T mindestens eine Proteinkonzentration CP eines Proteins oder die Konzentration von anderen Substanzen, welche in der Flüssigkeit gelöst sind oder als Suspensionen von Mikro- und Nanopartikeln oder von Zellen vorhanden sein können. Wie bereits beschrieben, können die Zusammenhänge zwischen der Temperatur T, der Viskosität und der Konzentration auch direkt in einem Speicher des Viskosimeters 1 oder in einem externen Speicher, auf welchen das Viskosimeter 1 zugreifen kann, abgelegt sein. In diesem Fall kann die Kontrolleinrichtung 8 des Viskosimeters 1 die Funktion der Auswerteeinheit 304 übernehmen und das Viskosimeter 1 um die Funktion eines Konzentrationsmessgerätes ergänzen.

Wie bereits erwähnt, ist das Viskosimeter 1 bei der Verwendung als Konzentrationsmessgerät, beziehungsweise das erfindungsgemässe Verfahren zur Bestimmung einer Konzentration einer Komponente in einem Fluid nicht auf die Messung von Proteinkonzentrationen in Flüssigkeiten beschränkt.

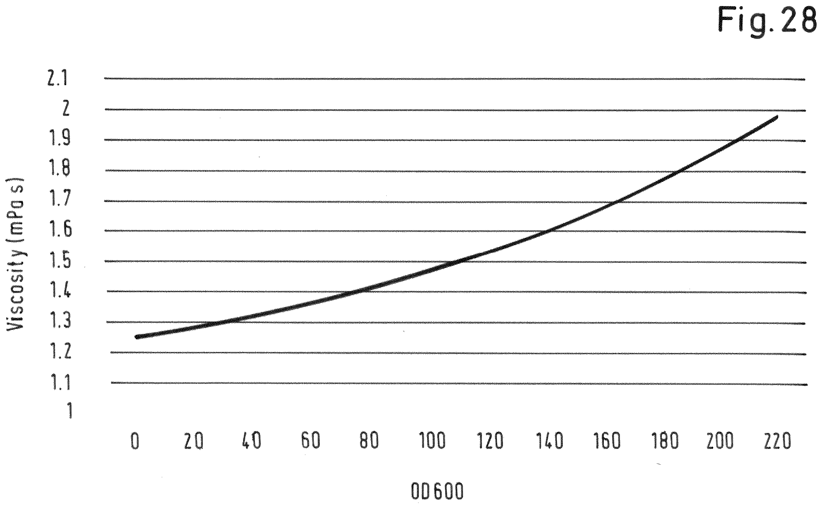

Ein anderes Anwendungsbeispiel ist die Bestimmung der Zelldichte in einer Zellsuspension (welche man auch als Zellkonzentration bezeichnen könnte). Als Beispiel für den Zusammenhang zwischen der Zelldichte in einer Zellsuspension und der Viskosität der Zellsuspension ist in Fig. 28 der Zusammenhang zwischen der Zelldichte von Escherichia-Coli Bakterien (E-Coli Bakterien) in einer Zellsuspension und der Viskosität dieser Zellsuspension (vertikale Achse) gezeigt.

Solche E-Coli Zellkulturen werden beispielsweise in Bioreaktoren zur Herstellung von Insulin, einem hochmolekularen Protein, verwendet. Als repräsentative Grösse für die Zelldichte ist in Fig. 28 auf der horizontalen Achse die optische Dichte der Zellsuspension bei einer Lichtwellenlänge von 600 nm aufgetragen, d.h. Fig. 28 zeigt den Zusammenhang zwischen der Viskosität der Zellsuspension und der optischen Dichte bei einer Lichtwellenlänge von 600 nm, dem sogenannten OD600-Wert. Da der Zusammenhang zwischen dem OD600-Wert und der Zelldichte für E-Coli Zellkulturen in sehr guter Näherung linear und zudem gut bekannt ist (ein OD600-Wert von 1 entspricht etwa 8*10^8 Zellen pro Milliliter), wird in der Biotechnologie häufig der OD600-Wert anstelle der Zelldichte angegeben. In Fig. 28 entspricht daher der maximale OD600-Wert von 220 etwa einer Zelldichte von 220*8*10^8 = 1.76*10^11 Zellen pro Milliliter. Das ist nahe an der Obergrenze der Zelldichten, welche mit E-Coli Zellkulturen erreicht werden können.

Mit Hilfe des zuvor beschriebenen Verfahrens lässt sich mit dem Viskosimeter 1 und der Auswerteeinheit 304, in welcher der in Fig. 28 dargestellte Zusammenhang zwischen der Viskosität der Zellsuspension und dem OD600-Wert als Datensatz gespeichert ist, in analoger Weise zur Funktion eines Konzentrationsmessgeräts die Funktion eines Zelldichtemessgeräts realisieren.

Die Bestimmung der Zelldichte mit dem Viskosimeter 1 ist allerdings im Vergleich zur Bestimmung mittels optischer Zelldichtemessgeräte üblicherweise weniger genau. Wie dies Fig. 28 zeigt, beträgt die Viskositätsänderung des Bioreaktormediums (Zellsuspension) über den gesamten Zelldichtebereich lediglich 0.75 mPa s. Dabei kann sich die Viskosität des Bioreaktormediums durch Temperatureinflüsse und die ändernde Zusammensetzung des Mediums um bis zu 20% ändern. Wenn also die Zelldichte aus der Viskositätsänderung bestimmt wird, resultiert als Folge der geringen Auflösung und der Fremdeinflüsse, welche nicht alle vollständig kompensiert werden können, eine im Vergleich zu optischen Verfahren ungenauere Messung. Bei tierischen Zellkulturen kann die Messung aufgrund der geringeren Zelldichten noch ungenauer werden.

Somit ist die praktische Bedeutung der Bestimmung der Zelldichte mittels des Viskosimeters 1 beschränkt. Hingegen lässt sich aus dem in Fig. 28 dargestellten Zusammenhang zwischen der Viskosität und dem OD600-Wert, die bei der E-Coli Zellkultur mit zunehmender Zelldichte zu erwartende Viskositätsänderung mit sehr guter Auflösung und hoher Genauigkeit aus dem OD600-Wert bestimmen. In diesem Fall ist es von Vorteil, dass der Einfluss der Zelldichte auf die Viskosität gering und dadurch die Zelldichte-Viskositäts-Kurve relativ flach ist.

Im Unterschied zur Abhängigkeit der Viskosität der Zellbrühe von der Zelldichte ist die Abhängigkeit der Viskosität von der Proteinkonzentration in der Zellbrühe deutlich stärker. Durch Kenntnis des Viskositätsbeitrages der Zelldichte zu der Gesamtviskosität der Zellbrühe lässt sich in Bioteaktoren die extrazelluläre Proteinkonzentration bestimmen, sofern die Viskosität der Zellbrühe und die zu erwartende Proteinzusammensetzung bekannt sind.

Dazu wird die Viskosität der Zellbrühe gemessen, über den OD600-Wert und den in Fig. 28 dargestellten Zusammenhang zwischen der Viskosität und dem OD600-Wert der Einfluss der Zellkonzentration auf die Viskosität bestimmt und mit der Viskosität der Zellbrühe verrechnet. Besonders interessant kann dieses Verfahren für die Bestimmung der Konzentration von sogenannten "Leakage Proteinen" sein, welche durch Zell Lyse (Aufbrechen der Zellmembran) aus den kultivierten Zellen in die Zellbrühe gelangen. Die Konzentration dieser Leakage Proteine ist unter anderem ein Indikator für den Anteil der produktiven Zellen, der Cell-Viability, und gibt darüber Aufschluss, ob noch genügend produktive Zellen vorhanden sind, welche den gewünschten Wirkstoff produzieren. Gewisse Leakage Proteine können auf die Zellen eine toxische Wirkung haben und dadurch ein Absterben der Zellen beschleunigen. Solche toxischen Proteine müssen später im sogenannten "Downstream Processing" mit teuren Prozessen (z.B. Chromatographie) vom Wirkstoff getrennt werden. Deshalb ist es wichtig, möglichst genau den richtigen Zeitpunkt bestimmen zu können, wann die Zellkultur abgebrochen werden soll. Die Konzentration der Leakage Proteine ist dafür ein wichtiger Indikator.

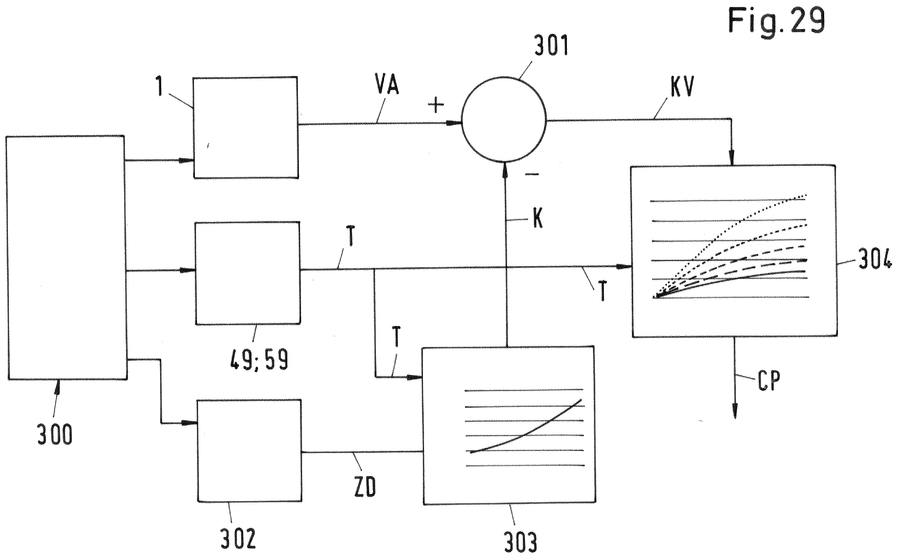

Fig. 29 zeigt eine schematische Darstellung eines zweiten Ausführungsbeispiels des erfindungsgemässen Verfahrens. Dieses zweiten Ausführungsbeispiel dient zur Messung der Konzentration von extrazellulären Proteinen, insbesondere von Leakage Proteinen, in Bioreaktoren. Bei diesem zweiten Ausführungsbeispiel wird zumindest eine Proteinkonzentration in einer Zellbrühe ermittelt. In Fig. 29 ist ein Bioreaktor 300 gezeigt, in welchem eine Zellbrühe für einen biologischen bzw. einen biotechnologischen Prozess enthalten ist. Der Bioreaktor 300 ist üblicherweise in ein Fluidsystem integriert, welches Filtervorrichtungen, Pumpen, Zuführungen, beispielsweise für eine Nährlösung, Entnahmevorrichtungen umfassen kann, was in Fig. 29 nicht näher dargestellt ist. Das Viskosimeter 1 ist für eine Inline-Messung ausgestaltet, das heisst die Messvorrichtung 10 des Viskosimeters 1 wird kontinuierlich von der Zellbrühe durchströmt. Mittels des Viskosimeters 1 wird kontinuierlich oder in vorgebbaren zeitlichen Abständen die aktuelle Viskosität VA der Zellbrühe bestimmt, welche einer Korrektureinheit 301 übermittelt wird. Mittels des Temperatursensors 49 und/oder 59 wird die Temperatur T der Zellbrühe ermittelt. Auch wenn die Temperatur T vorzugsweise mit einem in dem Viskosimeter 1 angeordneten Temperatursensor bestimmt wird, also beispielsweise mit dem Temperatursensor 49 in der Messvorrichtung 10 oder mit dem Temperatursensor 59 in der Antriebsvorrichtung 50, so ist es auch möglich, die Temperatur T mit einer externen Temperaturmessvorrichtung zu bestimmen, die nicht in das Viskosimeter 1 integriert ist.

Ferner wird in einer Messvorrichtung 302 eine aktuelle Zelldichte ZD in der Zellbrühe ermittelt. Die Zelldichte ZD wird vorzugsweise photometrisch ermittelt. Wie bereits beschrieben ist es üblich, die Zelldichte in einem biologischen Fluid, also hier in der Zellbrühe, durch den OD600-Wert zu charakterisieren, da dieser einfach messbar ist und gut mit der Zelldichte korreliert. Der OD600-Wert gibt die optische Dichte der Zellbrühe bei einer Lichtwellenlänge von 600 nm an. Aus dieser kann dann die aktuelle Zelldichte ZD ermittelt werden.

In einem Korrekturmodul 303 wird anhand eines Referenzwertes für den Zusammenhang zwischen der Zelldichte ZD beziehungsweise dem OD600-Wert und der Viskosität für die gemessene Temperatur T ein Korrekturwert K für die Viskosität bei der Temperatur T bestimmt. Der Korrekturwert K wird an die Korrektureinheit 301 übermittelt. Als Referenzwert kann in dem Korrekturmodul 303 beispielsweise der Zusammenhang zwischen der Zelldichte beziehungsweise dem OD600-Wert und der Viskosität in der frischen Zellsuppe verwendet werden. Mit dem Begriff "frische Zellsuppe" ist damit die Zellsuppe gemeint, in der noch keine oder nur geringe Konzentrationen von extrazellulären Proteinen vorhanden sind und noch keine oder nur wenige Proteine, die beim Zerstören bzw. Aufplatzen der Zellen in die Zellbrühe übergehen (leaked protein). In der Praxis variiert der Zeitraum, in welchem die Zellsuppe nach obiger Definition als frisch bezeichnet werden kann, mit der Art der gezüchteten Zellen und der Art der Zellkultur. Bei Batchkulturen von E-Coli Bakterien, kann die Zellsuppe nur etwa 24 bis 36 Stunden lang als "frisch" bezeichnet und für die Bestimmung des Referenzwertes herangezogen werden. Bei Zellkulturen von tierischen Zellen in Perfusionsbioreaktoren oder auch bei Fed-Batch Zellkulturen, kann die Zellsuppe nach obiger Definition manchmal mehrere Tage lang als "frisch" bezeichnet werden.

In der Korrektureinheit 301 wird die aktuelle Viskosität VA mit Hilfe des Korrekturwerts K korrigiert und eine korrigierte Viskosität KV ermittelt. Beispielsweise wird der Korrekturwert K von der aktuellen Viskosität VA abgezogen, um so die korrigierte Viskosität KV zu bestimmen. Die korrigierte Viskosität KV stellt also für die Temperatur den Wert der Viskosität unter Berücksichtigung der aktuellen Zelldichte ZD in der Zellbrühe dar. Die korrigierte Viskosität KV wird der Auswerteeinheit 304 zugeführt, in welcher solche Zusammenhänge zwischen der Temperatur T, der Viskosität und der Konzentration hinterlegt sind, wie sie beispielhaft in Fig. 26 gezeigt sind. Die Auswerteeinheit 304 bestimmt dann aus der korrigierten Viskosität KV und der Temperatur T mindestens eine Proteinkonzentration CP eines extrazellulären Proteins in der Zellsuppe.

## Patentansprüche

1. Konzentrationsmessgerät zur Inline Bestimmung einer Konzentration einer Komponente in einem Fluid mit einer Antriebsvorrichtung (50) und mit einer Messvorrichtung (10), wobei die Messvorrichtung (10) ein Messgehäuse (20) mit einem Einlass (21) und mit einem Auslass (22) für das Fluid umfasst, sowie eine Messkammer (23), in welcher ein Rotor (3) mit einem ring- oder scheibenförmigen magnetisch wirksamen Kern (31) vorgesehen ist, wobei die Antriebsvorrichtung (50) ein Antriebsgehäuse (60) umfasst, in welchem ein Stator (6) angeordnet ist, welcher mit dem Rotor (3) als elektromagnetischer Drehantrieb (90) zusammenwirkt, wobei der Stator (6) als Lager- und Antriebsstator ausgestaltet ist, mit welchem der Rotor (3) im Betriebszustand berührungslos magnetisch um eine axiale Richtung (A) antreibbar ist, und mit welchem der Rotor (3) berührungslos magnetisch bezüglich des Stators (6) lagerbar ist, und wobei im Antriebsgehäuse (60) ferner eine Kontrolleinrichtung (8) für die Ansteuerung des Stators (6) vorgesehen ist, **dadurch gekennzeichnet, dass** das Konzentrationsmessgerät eine Speichereinheit (40) aufweist, in welcher Kalibrierungsdaten für die Messvorrichtung (10) gespeichert sind, und das Konzentrationsmessgerät die Konzentration einer Komponente des Fluids anhand einer Betriebsgrösse des elektromagnetischen Drehantriebs (90) bestimmt.

2. Konzentrationsmessgerät nach Anspruch 1, wobei die Messvorrichtung (10) die Speichereinheit (40) umfasst, wobei die Antriebsvorrichtung (50) eine Schnittstelle (85) umfasst, über welche Daten aus der Speichereinheit (40) an die Kontrolleinheit (8) übermittelbar sind.

3. Konzentrationsmessgerät nach einem der vorangehenden Ansprüche, wobei die Messvorrichtung (10) gamma-sterilisierbar ausgestaltet ist, wobei bevorzugt die Speichereinheit (40) gammastabil ausgestaltet ist.

4. Konzentrationsmessgerät nach einem der vorangehenden Ansprüche, wobei die Messvorrichtung (10) einen Temperatursensor (49, 59) umfasst, mit welchem die Temperatur des Fluids in der Messvorrichtung ermittelbar ist.

5. Konzentrationsmessgerät nach einem der vorangehenden Ansprüche, wobei in der Kontrolleinrichtung (8) ein Zusammenhang zwischen der Konzentration und der Viskosität für eine und/oder mehrere Temperaturen hinterlegt sind, bevorzugterweise in Form einer Lookup-Tabelle.

6. Konzentrationsmessgerät nach einem der vorangehenden Ansprüche, wobei das Konzentrationsmessgerät einen zusätzlichen Speicher umfasst und/oder für die Kommunikation mit einem externen Speicher ausgestaltet ist, wobei in diesem Speicher ein und/oder mehrere Datensätze gespeichert sind, die einen Zusammenhang zwischen Viskosität, Temperatur und Konzentration einer und/oder mehrerer Flüssigkeiten und/oder Komponenten der Flüssigkeit beschreiben.

7. Konzentrationsmessgerät nach einem der vorangehenden Ansprüche, das eine Schnittstelle aufweist, wobei die Schnittstelle derart ausgestaltet ist, dass sie über eine Internet-Verbindung und/oder auswechselbare Speicher Zugriff auf Datensätze ermöglicht.

8. Konzentrationsmessgerät nach einem der vorangehenden Ansprüche, wobei die Betriebsgrösse ein Antriebsstrom und/oder ein Drehmoment, welches für das Antreiben der Rotation des Rotors benötigt wird, ist.

9. Konzentrationsmessgerät nach einem der vorangehenden Ansprüche, wobei das Konzentrationsmessgerät eine Auswerteeinheit (304) aufweist, wobei die Auswerteeinheit (304) über den Zusammenhang zwischen Antriebsstrom und/oder Drehmoment, und/oder Viskosität, und/oder Temperatur, und aus den gemessenen Antriebsstrom und/oder Drehmoment, und/oder gemessener Viskosität, und/oder gemessener Temperatur die Konzentration und/oder Konzentrationen bestimmt.

10. Konzentrationsmessgerät nach einem der vorangehenden Ansprüche, wobei das Konzentrationsmessgerät kontinuierlich und/oder in vorgebbaren zeitlichen Abständen eine aktuelle Viskosität (VA) des Fluids bestimmt.

11. Konzentrationsmessgerät nach einem der Ansprüche 9-10, wobei die Kontrolleinrichtung (8) die Auswerteeinheit (304) umfasst.

12. Konzentrationsmessgerät nach einem der vorangehenden Ansprüche, wobei eine Komponente des Fluids eine Zelldichte umfasst, wobei das Konzentrationsmessgerät die Zelldichte bestimmt oder die Zelldichte durch einen externen Sensor bestimmt wird, wobei die Zelldichte für die Bestimmung einer Konzentration einer Komponente in dem Fluid einbezogen wird.

13. Verfahren zur Inline Bestimmung einer Konzentration einer Komponente in einem Fluid umfassend die folgenden Schritte:
- Bereitstellen eines Konzentrationsmessgeräts, welches nach einem der Ansprüche 1-12 ausgestaltet ist,
- Bereitstellen eines Zusammenhangs zwischen mindestens der Konzentration der Komponente in dem Fluid und einer Betreibsgrösse des elektromagnetischen Drehantriebs (90) und/oder der Viskosität des Fluids,
- Bestimmen der Konzentration aus dem Zusammenhang zwischen mindestens der Konzentration und der Betriebsgrösse des elektromagnetischen Drehantriebs (90) und/oder der Viskosität des Fluids.

14. Verfahren nach Anspruch 13, wobei das Konzentrationsmessgerät relative Anteile in dem Fluid bestimmt, unter optionaler Zuhilfenahme anderer physikalischer Grössen wie beispielsweise der Ausbreitungsgeschwindigkeit von Ultraschall in der Flüssigkeit, der Absorption von Licht bei einer oder mehreren Wellenlängen oder des Refraktionsindexes von Licht bei einer oder mehreren Wellenlängen.

15. Verfahren nach Anspruch 13 - 14, wobei die Komponente des Fluids ein Protein und/oder eine Zusammensetzung mehrerer Proteine in einem Fluid ist, wobei die Proteine insbesondere Immunoglobuline sind, wobei für den Zusammenhang die Fluidtemperatur berücksichtigt wird.
